(19) Europäisches Patentamt
European Patent Office
Office européen des brevets

(11) EP 3 502 241 A1

# (12) EUROPEAN PATENT APPLICATION

(43) Date of publication:
**26.06.2019 Bulletin 2019/26**

(51) Int Cl.:
***C12N 9/02*** *(2006.01)* ***C12P 7/46*** *(2006.01)*

(21) Application number: **17209645.5**

(22) Date of filing: **21.12.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(83) **Declaration under Rule 32(1) EPC (expert solution)**

(71) Applicant: **BASF SE**
**67056 Ludwigshafen am Rhein (DE)**

(72) Inventors:
- **HOFF, Birgit**
  **67056 Ludwigshafen (DE)**
- **BOEHMER, Nico**
  **67056 Ludwigshafen (DE)**
- **HAEFNER, Stefan**
  **67056 Ludwigshafen (DE)**
- **SCHROEDER, Hartwig**
  **67056 Ludwigshafen (DE)**
- **ZELDER, Oskar**
  **67056 Ludwigshafen (DE)**
- **WITTMANN, Christoph**
  **66123 Saarbrücken (DE)**
- **SCHROER, Anna Christine**
  **67056 Ludwigshafen (DE)**

(74) Representative: **Herzog, Fiesser & Partner Patentanwälte PartG mbB**
**Dudenstrasse 46**
**68167 Mannheim (DE)**

## (54) MODIFIED MICROORGANISM FOR IMPROVED PRODUCTION OF SUCCINATE

(57) The present invention relates to a process of producing an organic compound, the process comprising I) cultivating a genetically modified microorganism in a culture medium comprising at least one assimilable carbon source to allow the genetically modified microorganism to produce the organic compound,
II) recovering the organic compound from the fermentation broth obtained in process step I), wherein the genetically modified microorganism comprises
A) at least one genetic modification that leads to an increased activity of the enzyme encoded by the *udhA*-gene, compared to the original microorganism that has not been genetically modified,

and wherein the original microorganism belongs to the family *Pasteurellaceae.*

The present invention also relates to a genetically modified microorganism and to the use thereof for the fermentative production of an organic compound from at least one assimilable carbon source.

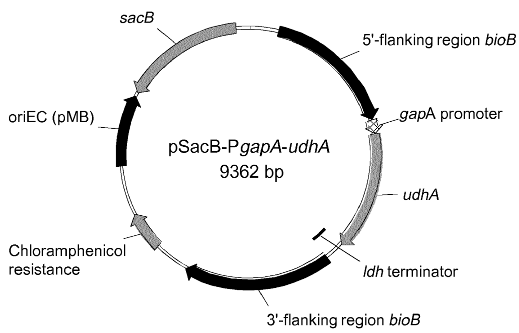


Fig. 1

EP 3 502 241 A1

## Description

The present invention relates to a process for producing an organic compound, preferably succinic acid, to a genetically modified microorganism and to the use of the genetically modified microorganism for the fermentative production of an organic compound, preferably succinic acid.

Organic compounds such as small dicarboxylic acids having 6 or fewer carbons are commercially significant chemicals with many uses. For example, the small diacids include 1,4-diacids, such as succinic acid, malic acid and tartaric acid, and the 5-carbon molecule itaconic acid. Other diacids include the two carbon oxalic acid, three carbon malonic acid, five carbon glutaric acid and the 6 carbon adipic acid and there are many derivatives of such diacids as well.

As a group the small diacids have some chemical similarity and their uses in polymer production can provide specialized properties to the resin. Such versatility enables them to fit into the downstream chemical infrastructure markets easily. For example, the 1,4-diacid molecules fulfill many of the uses of the large scale chemical maleic anhydride in that they are converted to a variety of industrial chemicals (tetrahydrofuran, butyrolactone, 1,4-butanediol, 2-pyrrolidone) and the succinate derivatives succindiamide, succinonitrile, diaminobutane and esters of succinate. Tartaric acid has a number of uses in the food, leather, metal and printing industries. Itaconic acid forms the starting material for production of 3-methylpyrrolidone, methyl-BDO, methyl-THF and others.

In particular, succinic acid or succinate - these terms are used interchangeably herein - has drawn considerable interest because it has been used as a precursor of many industrially important chemicals in the food, chemical and pharmaceutical industries. In fact, a report from the U.S. Department of Energy reports that succinic acid is one of 12 top chemical building blocks manufactured from biomass. Thus, the ability to make diacids in bacteria would be of significant commercial importance.

WO-A-2009/024294 discloses a succinic acid producing bacterial strain, being a member of the family of *Pasteurellaceae,* originally isolated from rumen, and capable of utilizing glycerol as a carbon source and variant and mutant strains derived there from retaining said capability, in particular, a bacterial strain designated DD1 as deposited with DSMZ (Deutsche Sammlung von Mikroorganismen und Zellkulturen GmbH, Inhoffenstr. 7B, D-38124 Braunschweig, Germany) having the deposit number DSM 18541 (ID 06-614) and having the ability to produce succinic acid. The DD1-strain belongs to the species *Basfia succiniciproducens* and the family of *Pasteurellaceae* as classified by Kuhnert et *al*., 2010. Mutations of these strains, in which the *ldhA*-gene and/or the *pflD*- or the *pflA*-gene have been disrupted, are disclosed in WO-A-2010/092155, these mutant strains being characterized by a significantly increased production of succinic acid from carbon sources such as glycerol or mixtures of glycerol and carbohydrates such as maltose, under anaerobic conditions compared to the DD1-wildtype disclosed in WO-A-2009/024294.

However, when using bacterial strains such as those disclosed in WO-A-2009/024294 or WO-A-2010/092155 for the production or organic compounds such as succinic acid, the yield of the desired organic compound is still improvable.

It was therefore an object of the present invention to overcome the disadvantages of the prior art.

In particular, it was an object of the present invention to provide a process for producing an organic compound such as succinic acid by means of which high yields of the desired organic compound can be achieved when using assimilable carbon sources such as glycerol, glucose, sucrose, xylose, lactose, fructose or maltose.

It was also an object of the present invention to provide a genetically modified microorganism that, compared to the original cell from which it has been derived by genetic modification, allows the production of an organic compound such as succinic acid from assimilable carbon sources such as glycerol, glucose, sucrose, xylose, lactose, fructose or maltose with higher yields.

A contribution to achieving the abovementioned aims is provided by a process of producing an organic compound, preferably succinic acid, the process comprising

I) cultivating a genetically modified microorganism in a culture medium comprising at least one assimilable carbon source to allow the genetically modified microorganism to produce the organic compound,

II) recovering the organic compound from the fermentation broth obtained in process step I),

wherein the genetically modified microorganism comprises

A) at least one genetic modification that leads to an increased activity of the enzyme encoded by the *udhA*-gene, compared to the original microorganism that has not been genetically modified,

and wherein the original microorganism belongs to the family *Pasteurellaceae.*

Surprisingly, it has been discovered that microorganisms of the family *Pasteurellaceae* comprising at least one genetic modification that leads to an increased activity of the enzyme encoded by the *udhA*-gene (encoding for a NAD(P)$^+$-transhydrogenase; EC 1.6.1.1) are characterized by an increased production of organic compounds such as succinic acid from assimilable carbon sources such as glycerol, glucose, sucrose, xylose, lactose, fructose or maltose, compared to the original microorganisms that have not been genetically modified.

In the process according to the present invention a genetically modified microorganism is used that comprises

A) at least one genetic modification that leads to an increased activity of the enzyme encoded by the *udhA*-gene, compared to the original microorganism that has not been genetically modified, wherein the original microorganism belongs to the family *Pasteurellaceae.*

Such a genetically modified microorganism is obtainable by a process at least comprising the process steps:

i) providing an original microorganism of the family *Pasteurellaceae*;

ii) genetically modifying the microorganism in such a way that the activity of the enzyme encoded by the *udhA*-gene is increased;

iii) optionally performing further genetic modifications of the microorganism that lead to an increased or reduced activity of one or more further enzymes being different from the enzyme encoded by the *udhA*-gene, wherein process step iii) can be performed at any time after process step i) (particularly before process step ii), after process step ii) or before and after process step ii)).

The term *"original microorganism"* as used herein preferably refers to the so called *"wildtype"*-strain. The term *"wildtype"* refers to a microorganism whose genome, in particular whose *udhA*-gene and whose regulatory elements of the *udhA*-gene, is/are present in a state as generated naturally as the result of evolution. Particularly, the term *"wildtype"* refers to a microorganism of the family *Pasteurellaceae* that does not express a soluble transhydrogenase encoded by the *udhA*-gene of *E. coli.* As a consequence, the term *"wildtype"* preferably does not cover those microorganisms whose gene sequences have at least in part been modified by man by means of recombinant methods. The term *"genetically modified microorganism"* thus includes a microorganism which has been genetically altered, modified or engineered (e.g., genetically engineered) such that it exhibits an altered, modified or different genotype and/or phenotype (e. g., when the genetic modification affects coding nucleic acid sequences of the microorganism) as compared to the naturally-occurring wildtype microorganism from which it was derived. According to a particular preferred embodiment of the genetically modified microorganism used in the process of the present invention the genetically modified microorganism is a recombinant microorganism, which means that the microorganism has been obtained using recombinant DNA. The expression *"recombinant DNA"* as used herein refers to DNA sequences that result from the use of laboratory methods (molecular cloning) to bring together genetic material from multiple sources, creating sequences that would not otherwise be found in biological organisms. An example of such a recombinant DNA is a plasmid into which a heterologous DNA-sequence has been inserted.

The original microorganism from which the genetically modified microorganism has been derived by the above described genetic modification belongs to the family *Pasteurellaceae. Pasteurellaceae* comprise a large family of Gram-negative Proteobacteria with members ranging from bacteria such as *Haemophilus influenzae* to commensals of the animal and human mucosa. Most members live as commensals on mucosal surfaces of birds and mammals, especially in the upper respiratory tract. *Pasteurellaceae* are typically rod-shaped, and are a notable group of facultative anaerobes. They can be distinguished from the related *Enterobacteriaceae* by the presence of oxidase, and from most other similar bacteria by the absence of flagella. Bacteria in the family *Pasteurellaceae* have been classified into a number of genera based on metabolic properties and there sequences of the 16S RNA and 23S RNA. Many of the *Pasteurellaceae* contain pyruvate-formate-lyase genes and are capable of anaerobically fermenting carbon sources to organic acids.

In this context it is particularly preferred that the original microorganism belongs to the genus *Basfia* and it is particularly preferred that it belongs to the species *Basfia succiniciproducens.*

Most preferably, the original microorganism is *Basfia succiniciproducens*-strain DD1 deposited under the Budapest Treaty with DSMZ (Deutsche Sammlung von Mikroorganismen und Zellkulturen, GmbH), Germany, having the deposit number DSM 18541. This strain has been originally isolated from the rumen of a cow of German origin. *Pasteurella* bacteria can be isolated from the gastro-intestinal tract of animals and, preferably, mammals. The bacterial strain DD1, in particular, can be isolated from bovine rumen and is capable of utilizing glycerol (including crude glycerol) as a carbon source. Further strains of the genus *Basfia* that can be used for preparing the modified microorganism according to the present invention are the *Basfia*-strain that has been deposited under the deposit number DSM 22022 or the *Basfia*-strains that have been deposited with the Culture Collection of the University of Göteborg (CCUG), Sweden, having the deposit numbers CCUG 57335, CCUG 57762, CCUG 57763, CCUG 57764, CCUG 57765 or CCUG 57766. Said strains have been originally isolated from the rumen of cows of German or Swiss origin.

In this context it is particularly preferred that the original microorganism has a 16S rDNA of **SEQ ID NO: 1** or a sequence, which shows a sequence homology of at least 96 %, at least 97 %, at least 98 %, at least 99 % or at least 99.9% with **SEQ ID NO: 1.** It is also preferred that the original microorganism has a 23S rDNA of **SEQ ID NO: 2** or a sequence, which shows a sequence homology of at least 96 %, at least 97 %, at least 98 %, at least 99 % or at least 99.9 % with **SEQ ID NO: 2.**

The identity in percentage values referred to in connection with the various polypeptides or polynucleotides to be used for the modified microorganism according to the present invention is, preferably, calculated as identity of the residues over the complete length of the aligned sequences, such as, for example, the identity calculated (for rather similar sequences) with the aid of the program needle from the bioinformatics software package EMBOSS (Version 5.0.0, http://emboss.source-forge.net/what/) with the default parameters which are, i.e. gap open (penalty to open a gap): 10.0, gap extend (penalty to extend a gap): 0.5, and data file (scoring matrix file included in package): EDNAFUL.

It should be noted that the original microorganism from which the genetically modified microorganism has been derived is not limited to one of the above mentioned strains, especially not to *Basfia succiniciproducens*-strain DD1, but may also comprise variants of these strains. In this context the expression "*a variant of a strain*" comprises every strain having the same or essentially the same characteristics as the wildtype-strain. In this context it is particularly preferred that the 16 S rDNA of the variant has an identity of at least 90 %, preferably at least 95 %, more preferably at least 99 %, more preferably at least 99.5 %, more preferably at least 99.6 %, more preferably at least 99.7 %, more preferably at least 99.8 % and most preferably at least 99.9 % with the wildtype from which the variant has been derived. It is also particularly preferred that the 23 S rDNA of the variant has an identity of at least 90 %, preferably at least 95 %, more preferably at least 99 %, more preferably at least 99.5 %, more preferably at least 99.6 %, more preferably at least 99.7 %, more preferably at least 99.8 % and most preferably at least 99.9 % with the wildtype from which the variant has been derived. A variant of a strain in the sense of this definition can, for example, be obtained by treating the wildtype-strain with a mutagenizing chemical agent, X-rays, or UV light.

According to a preferred embodiment of the genetically modified microorganism the *udhA*-gene comprises a nucleic acid selected from the group consisting of:

a1) nucleic acids having the nucleotide sequence of **SEQ ID NO: 3**;

b1) nucleic acids encoding the amino acid sequence of **SEQ ID NO: 4**;

c1) nucleic acids which are at least 70%, at least 80 %, at least 85 %, at least 90 %, at least 95 %, at least 96 %, at least 97 %, at least 98 %, at least 99 %, at least 99.5 %, at least 99.6 %, at least 99.7 %, at least 99.8 % or at least 99.9 %, most preferably 100 % identical to the nucleic acid of a1) or b1), the identity being the identity over the total length of the nucleic acids of a1) or b1);

d1) nucleic acids encoding an amino acid sequence which is at least 70%, at least 80 %, at least 85 %, at least 90 %, at least 95 %, at least 96 %, at least 97 %, at least 98 %, at least 99 %, at least 99.5 %, at least 99.6 %, at least 99.7 %, at least 99.8 % or at least 99.9 %, most preferably 100 % identical to the amino acid sequence encoded by the nucleic acid of a1) or b1), the identity being the identity over the total length of amino acid sequence encoded by the nucleic acids of a1) or b1);

e1) nucleic acids capable of hybridizing under stringent conditions with a complementary sequence of any of the nucleic acids according to a1) or b1); and

f1) nucleic acids encoding the same protein as any of the nucleic acids of a1) or b1), but differing from the nucleic acids of a1) or b1) above due to the degeneracy of the genetic code.

The term *"hybridization"* as used herein includes *"any process by which a strand of nucleic acid molecule joins with a complementary strand through base pairing"* (J. Coombs (1994) Dictionary of Biotechnology, Stockton Press, New York). Hybridization and the strength of hybridization (i.e., the strength of the association between the nucleic acid molecules) is impacted by such factors as the degree of complementarity between the nucleic acid molecules, stringency of the conditions involved, the Tm of the formed hybrid, and the G:C ratio within the nucleic acid molecules.

As used herein, the term *"Tm"* is used in reference to the *"melting temperature".* The melting temperature is the temperature at which a population of double-stranded nucleic acid molecules becomes half dissociated into single strands. The equation for calculating the Tm of nucleic acid molecules is well known in the art. As indicated by standard references, a simple estimate of the Tm value may be calculated by the equation: Tm = 81.5 + 0.41(% G+C), when a nucleic acid molecule is in aqueous solution at 1 M NaCl (see e.g., Anderson and Young, Quantitative Filter Hybridization, in Nucleic Acid Hybridization (1985)). Other references include more sophisticated computations, which take structural as well as sequence characteristics into account for the calculation of Tm. Stringent conditions, are known to those skilled in the art and can be found in Current Protocols in Molecular Biology, John Wiley & Sons, N.Y. (1989), 6.3.1-6.3.6.

In particular, the term *"stringency conditions"* refers to conditions, wherein 100 contiguous nucleotides or more, 150 contiguous nucleotides or more, 200 contiguous nucleotides or more or 250 contiguous nucleotides or more which are a fragment or identical to the complementary nucleic acid molecule (DNA, RNA, ssDNA or ssRNA) hybridizes under conditions equivalent to hybridization in 7% sodium dodecyl sulfate (SDS), 0.5 M NaPO4, 1 mM EDTA at 50°C with washing in 2 × SSC, 0.1% SDS at 50°C or 65°C, preferably at 65°C, with a specific nucleic acid molecule (DNA; RNA, ssDNA or ssRNA). Preferably, the hybridizing conditions are equivalent to hybridization in 7% sodium dodecyl sulfate (SDS), 0.5 M NaPO4, 1 mM EDTA at 50°C with washing in 1 × SSC, 0.1% SDS at 50°C or 65°C, preferably 65°C, more preferably the hybridizing conditions are equivalent to hybridization in 7% sodium dodecyl sulfate (SDS), 0.5 M Na-PO4, 1 mM EDTA at 50°C with washing in 0.1 × SSC, 0.1% SDS at 50°C or 65°C, preferably 65°C. Preferably, the complementary nucleotides hybridize with a fragment or the whole *fruA* nucleic acids. Alternatively, preferred hybridization conditions encompass hybridization at 65°C in 1 × SSC or at 42°C in 1 × SSC and 50% formamide, followed by washing at 65°C in 0.3 × SSC or by hybridization at 50°C in 4 × SSC or at 40°C in 6 × SSC and 50% formamide, followed by washing at 50°C in 2 × SSC. Further preferred hybridization conditions are 0.1 % SDS, 0.1 SSD and 65°C.

Nucleic acid having the nucleotide sequence of **SEQ ID NO: 3** corresponds to the *udhA*-gene of *E. coli.* This gene encodes to a soluble (i. e. not membrane-bound) transhydrogenase.

Increased NAD(P)$^+$-transhydrogenase-activity

The genetically modified microorganisms that is used in the process according to the present invention comprises A) at least one genetic modification that leads to an increased activity of the enzyme encoded by the *udhA*-gene, compared to the original microorganism that has not been genetically modified. Such a genetic modification can, for example, be a modification of the *udhA*-gene itself and/or a modification of a regulatory element of the *udhA*-gene, wherein the modification of the *udhA*-gene and/or the modification of a regulatory element of the *udhA*-gene lead/leads to an increased activity of the enzyme encoded by the *udhA*-gene, compared to the original microorganism in which the *udhA*-gene and/or the regulatory element of the *udhA*-gene have/has not been modified.

The increase of the enzyme activity ($\Delta_{\text{activity}}$) is - in case of an original microorganism which already has a certain activity of NAD(P)$^+$-transhydrogenase - preferably defined as follows:

$$\Delta_{\text{activity}} = \left( \frac{\text{activity of the genetically modified microorganism}}{\text{activity of the original microorganism}} \times 100\% \right) - 100\%$$

wherein, when determining $\Delta_{\text{activity}}$, the activity in the original microorganism and the activity in the modified microorganism are determined under exactly the same conditions. The activity of NAD(P)$^+$-transhydrogenase that is encoded by the *udhA*-gene can be determined by measuring the intracellular NADH/NAD$^+$- and NADPH/NADP$^+$-ratios as disclosed by Yamauchi et al.: "Enhanced acetic acid and succinic acid production under microaerobic conditions by Corynebacterium glutamicum harboring Escherichia coli transhydrogenase gene pntAB"; J. Gen. Appl. Microbiol. (2014), Vol. 60, p. 112-118.

The increased activity of NAD(P)$^+$-transhydrogenase can be an increase of the enzymatic activity by 1 to 10000%, compared to the activity of said enzyme in the wildtype of the microorganism, or an increase of the enzymatic activity by at least 50 %, or at least 100 %, or at least 200 %, or at least 300 %, or at least 400 %, or at least 500 %, or at least 600 % or at least 700 %, or at least 800 %, or at least 900 %, or at least 1000 %, or at least 5000 %. Preferably, the increase of the activity of an enzyme is in the range of 10 to 1000 %, more preferably in the range of 100 to 500 %.

An increased NAD(P)$^+$-transhydrogenase can be accomplished by a genetic modification of the *udhA*-gene itself, for example by increasing the copy number of the *udhA*-gene, by using a gene or allele which codes for a corresponding enzyme having an increased activity or by introducing one or more gene mutations which lead to an increased activity of NAD(P)$^+$-transhydrogenase. Such mutations can again be generated undirected either by classical methods, such as evolutionary adoption, UV irradiation or mutagenic chemicals, or targeted by genetic engineering methods such as deletion (s), insertion (s) and / or nucleotide exchange(s) by side directed mutagenesis. Also, an increased NAD(P)$^+$-transhydrogenase-activity can be accomplished by a modification of regulatory elements of the *udhA*-gene, such as a genetic modification of the *udhA*-promoter sequence or a modification of regulatory proteins, suppressors, enhancers, transcriptional activators and the like involved in transcription of the *udhA*-gene and/or the translation of the gene product. In this context it is, for example, possible to use a promoter sequence that, compared to the promoter sequence in the original microorganism, is stronger. It is, of course, also possible to combine these measures in order to increase the NAD(P)$^+$-transhydrogenase-activity.

According to the invention, genetically modified microorganisms are produced, for example by transformation, transduction, conjugation, or a combination of these methods, with a vector containing the desired gene, an allele of this gene or parts thereof, and includes a gene enabling the expression of the vector. The heterologous expression is achieved in particular by integrating the gene or alleles into the chromosome of the cell or an extrachromosomally replicating vector.

In this context it is particularly preferred that the at least one genetic modification A) comprises an overexpression of the *udhA*-gene, preferably an overexpression of the *udhA*-gene under the control of the *pckA*-promoter or under the control of the *gapA*-promoter. The *udhA*-gene that is overexpressed can either be located on an episomal plasmid in which it is preferably arranged under the control of the *pckA*-promoter or the *gapA*-promoter, or the *udhA*-gene can be integrated into the chromosome of a microorganism that may already comprise either a natural copy of the *udhA*-gene encoding for a soluble transhydrogenase or - as it is the case in the *Basfia*-strains that are described herein - a natural copy of the *pntA*-gene and/or the *pntB*-gene encoding for a membrane-bound transhydrogenase catalyzing the same reactions as the transhydrogenase encoded by the *udhA*-gene, wherein an integration of the *udhA*-gene into the chromosome of the microorganism under the control of the *pckA*-promoter or the *gapA*-rpmoter is again particularly preferred.

The *pckA*-promoter preferably comprises a nucleic acid selected from the group consisting of:

a2) nucleic acids having the nucleotide sequence of **SEQ ID NO: 5**;

b2) nucleic acids which are at least 70%, at least 80 %, at least 85 %, at least 90 %, at least 95 %, at least 96 %, at least 97 %, at least 98 %, at least 99 %, at least 99.5 %, at least 99.6 %, at least 99.7 %, at least 99.8 % or at least 99.9 %, most preferably 100 % identical to the nucleic acid of a2), the identity being the identity over the total length of the nucleic acids of a2).

The *gapA*-promoter preferably comprises a nucleic acid selected from the group consisting of:

a3) nucleic acids having the nucleotide sequence of **SEQ ID NO: 6**;

b3) nucleic acids which are at least 70%, at least 80 %, at least 85 %, at least 90 %, at least 95 %, at least 96 %, at least 97 %, at least 98 %, at least 99 %, at least 99.5 %, at least 99.6 %, at least 99.7 %, at least 99.8 % or at least 99.9 %, most preferably 100 % identical to the nucleic acid of a3), the identity being the identity over the total length of the nucleic acids of a3).

The extent in which a gene is expressed in a cell can, for example, be determined by means of Real-Time PCR. Details for determining gene expression in a cell can by means of Real-Time PCT are disclosed by Wong and Medrano in "Real-time PCR for mRNA quantitation", BioTechniques, Vol. 39, No. 1, July 2005, pp. 75-85.

In the following, a suitable technique for recombination, in particular for introducing a mutation or for deleting sequences, is described.

This technique is also sometimes referred to as the "Campbell recombination" herein (Leenhouts et al., Appl Env Microbiol. (1989), Vol. 55, pages 394-400). "Campbell in", as used herein, refers to a transformant of an original host cell in which an entire circular double stranded DNA molecule (for example a plasmid) has integrated into a chromosome by a single homologous recombination event (a cross in event), and that effectively results in the insertion of a linearized version of said circular DNA molecule into a first DNA sequence of the chromosome that is homologous to a first DNA sequence of the said circular DNA molecule. "Campbelled in" refers to the linearized DNA sequence that has been integrated into the chromosome of a "Campbell in" transformant. A "Campbell in" contains a duplication of the first homologous DNA sequence, each copy of which includes and surrounds a copy of the homologous recombination crossover point.

"Campbell out", as used herein, refers to a cell descending from a "Campbell in" transformant, in which a second homologous recombination event (a cross out event) has occurred between a second DNA sequence that is contained on the linearized inserted DNA of the "Campbelled in" DNA, and a second DNA sequence of chromosomal origin, which is homologous to the second DNA sequence of said linearized insert, the second recombination event resulting in the deletion (jettisoning) of a portion of the integrated DNA sequence, but, importantly, also resulting in a portion (this can be as little as a single base) of the integrated Campbelled in DNA remaining in the chromosome, such that compared to the original host cell, the "Campbell out" cell contains one or more intentional changes in the chromosome (for example, a single base substitution, multiple base substitutions, insertion of a heterologous gene or DNA sequence, insertion of an additional copy or copies of a homologous gene or a modified homologous gene, or insertion of a DNA sequence comprising more than one of these aforementioned examples listed above). A "Campbell out" cell is, preferably, obtained by a counter-selection against a gene that is contained in a portion (the portion that is desired to be jettisoned) of the "Campbelled in" DNA sequence, for example the *Bacillus subtilis sacB*-gene, which is lethal when expressed in a cell that is grown in the presence of about 5% to 10% sucrose. Either with or without a counter-selection, a desired "Campbell out" cell can be obtained or identified by screening for the desired cell, using any screenable phenotype, such as, but not limited to, colony morphology, colony color, presence or absence of antibiotic resistance, presence or absence of a given DNA sequence by polymerase chain reaction, presence or absence of an auxotrophy, presence or absence of an enzyme, colony nucleic acid hybridization, antibody screening, etc. The term "Campbell in" and "Campbell out" can also be used as verbs in various tenses to refer to the method or process described above.

It is understood that the homologous recombination events that leads to a "Campbell in" or "Campbell out" can occur over a range of DNA bases within the homologous DNA sequence, and since the homologous sequences will be identical to each other for at least part of this range, it is not usually possible to specify exactly where the crossover event occurred. In other words, it is not possible to specify precisely which sequence was originally from the inserted DNA, and which was originally from the chromosomal DNA. Moreover, the first homologous DNA sequence and the second homologous DNA sequence are usually separated by a region of partial non-homology, and it is this region of non-homology that remains deposited in a chromosome of the "Campbell out" cell.

Preferably, first and second homologous DNA sequence are at least about 200 base pairs in length, and can be up to several thousand base pairs in length. However, the procedure can be made to work with shorter or longer sequences. For example, a length for the first and second homologous sequences can range from about 500 to 2000 bases, and the obtaining of a "Campbell out" from a "Campbell in" is facilitated by arranging the first and second homologous sequences to be approximately the same length, preferably with a difference of less than 200 base pairs and most preferably with the shorter of the two being at least 70% of the length of the longer in base pairs.

According to a further preferred embodiment of the genetically modified microorganism that is used in the process according to the present invention the microorganism may additionally be characterized by

- a reduced pyruvate formate lyase activity,
- a reduced lactate dehydrogenase activity, and/or
- a reduced pyruvate formate lyase activity and a reduced lactate dehydrogenase activity.

Modified microorganisms being deficient in lactate dehydrogenase and/or being deficient in pyruvate formate lyase activity are disclosed in WO-A-2010/092155, US 2010/0159543 and WO-A-2005/052135, the disclosure of which with respect to the different approaches of reducing the activity of lactate dehydrogenase and/or pyruvate formate lyase in a microorganism, preferably in a bacterial cell of the genus *Pasteurella,* particular preferred in *Basfia succiniciproducens* strain DD1, is incorporated herein by reference. Methods for determining the pyruvate formate lyase activity are, for example, disclosed by Asanum N. and Hino T. in "Effects of pH and Energy Supply on Activity and Amount of Pyruvate-Formate-Lyase in Streptococcus bovis", Appl. Environ. Microbiol. (2000), Vol. 66, pages 3773-3777 and methods for determining the lactate dehydrogenase activity are, for example, disclosed by Bergmeyer, H.U., Bergmeyer J. and Grassl, M. (1983-1986) in "Methods of Enzymatic Analysis", 3rd Edition, Volume III, pages 126-133, Verlag Chemie, Weinheim

In this context it is preferred that the reduction of the activity of lactate dehydrogenase is achieved by an inactivation of the *Idha*-gene (which encodes the lactate dehydrogenase; LdhA; EC 1.1.1.27 or EC 1.1.1.28) and the reduction of the pyruvate formate lyase is achieved by an inactivation of the *pflA*-gene (which encodes for an activator of pyruvate formate lyase; PflA; EC 1.97.1.4) or the *pflD*-gene (which encodes the pyruvate formate lyase; PflD; EC 2.3.1.54), wherein the inactivation of these genes (i. e. *IdhA, pflA* and *pflD*) is preferably achieved by a deletion of theses genes or parts thereof, by a deletion of a regulatory element of these genes or at least a part thereof or by an introduction of at least one mutation into these genes, wherein these modifications are preferably performed by means of the "Campbell recombination" as described above.

The *IdhA*-gene the activity of which is reduced in the genetically modified microorganism that is used in the process according to the present invention preferably comprises a nucleic acid selected from the group consisting of:

$\alpha$1) nucleic acids having the nucleotide sequence of **SEQ ID NO: 7**;

$\alpha$2) nucleic acids encoding the amino acid sequence of **SEQ ID NO: 8**;

$\alpha$3) nucleic acids which are at least 70 %, at least 80 %, at least 85 %, at least 90 %, at least 95 %, at least 96 %, at least 97 %, at least 98 %, at least 99 %, at least 99.5 %, at least 99.6 %, at least 99.7 %, at least 99.8 % or at least 99.9 %, most preferably 100 % identical to the nucleic acid of $\alpha$1) or $\alpha$2), the identity being the identity over the total length of the nucleic acids of $\alpha$1) or $\alpha$2);

α4) nucleic acids encoding an amino acid sequence which is at least 70 %, at least 80 %, at least 85 %, at least 90 %, at least 95 %, at least 96 %, at least 97 %, at least 98 %, at least 99 %, at least 99.5 %, at least 99.6 %, at least 99.7 %, at least 99.8 % or at least 99.9 %, most preferably 100 % identical to the amino acid sequence encoded by the nucleic acid of α1) or α2), the identity being the identity over the total length of amino acid sequence encoded by the nucleic acids of α1) or α2);

α5) nucleic acids capable of hybridizing under stringent conditions with a complementary sequence of any of the nucleic acids according to α1) or α2); and

α6) nucleic acids encoding the same protein as any of the nucleic acids of α1) or α2), but differing from the nucleic acids of α1) or α2) above due to the degeneracy of the genetic code.

The *pflA*-gene the activity of which is reduced in the genetically modified microorganism that is used in the process according to the present invention preferably comprises a nucleic acid selected from the group consisting of:

β1) nucleic acids having the nucleotide sequence of **SEQ ID NO: 9**;

β2) nucleic acids encoding the amino acid sequence of **SEQ ID NO: 10**;

β3) nucleic acids which are at least 70 %, at least 80 %, at least 85 %, at least 90 %, at least 95 %, at least 96 %, at least 97 %, at least 98 %, at least 99 %, at least 99.5 %, at least 99.6 %, at least 99.7 %, at least 99.8 % or at least 99.9 %, most preferably 100 % identical to the nucleic acid of β1) or β2), the identity being the identity over the total length of the nucleic acids of β1) or β2);

β4) nucleic acids encoding an amino acid sequence which is at least 70 %, at least 80 %, at least 85 %, at least 90 %, at least 95 %, at least 96 %, at least 97 %, at least 98 %, at least 99 %, at least 99.5 %, at least 99.6 %, at least 99.7 %, at least 99.8 % or at least 99.9 %, most preferably 100 % identical to the amino acid sequence encoded by the nucleic acid of β1) or β2), the identity being the identity over the total length of amino acid sequence encoded by the nucleic acids of β1) or β2)

β5) nucleic acids capable of hybridizing under stringent conditions with a complementary sequence of any of the nucleic acids according to β1) or β2); and

β6) nucleic acids encoding the same protein as any of the nucleic acids of β1) or β2), but differing from the nucleic acids of β1) or β2) above due to the degeneracy of the genetic code.

The *pflD*-gene the activity of which is reduced in the genetically modified microorganism that is used in the process according to the present invention preferably comprises a nucleic acid selected from the group consisting of:

γ1) nucleic acids having the nucleotide sequence of **SEQ ID NO: 11**;

γ2) nucleic acids encoding the amino acid sequence of **SEQ ID NO: 12**;

γ3) nucleic acids which are at least 70 %, at least 80 %, at least 85 %, at least 90 %, at least 95 %, at least 96 %, at least 97 %, at least 98 %, at least 99 %, at least 99.5 %, at least 99.6 %, at least 99.7 %, at least 99.8 % or at least 99.9 %, most preferably 100 % identical to the nucleic acid of γ1) or γ2), the identity being the identity over the total length of the nucleic acids of γ1) or γ2);

γ4) nucleic acids encoding an amino acid sequence which is at least 70 %, at least 80 %, at least 85 %, at least 90 %, at least 95 %, at least 96 %, at least 97 %, at least 98 %, at least 99 %, at least 99.5 %, at least 99.6 %, at least 99.7 %, at least 99.8 % or at least 99.9 %, most preferably 100 % identical to the amino acid sequence encoded by the nucleic acid of γ1) or γ2), the identity being the identity over the total length of amino acid sequence encoded by the nucleic acids of γ1) or γ2);

γ5) nucleic acids capable of hybridizing under stringent conditions with a complementary sequence of any of the nucleic acids according to γ1) or γ2); and

γ6) nucleic acids encoding the same protein as any of the nucleic acids of γ1) or γ2), but differing from the nucleic acids of γ1) or γ2) above due to the degeneracy of the genetic code.

In this context it is preferred that the modification of the genetically modified microorganism that is used in the process according to the present invention, in addition to the at least one modification A), further comprises:

B) a deletion of the *ldha*-gene or at least a part thereof, a deletion of a regulatory element of the *ldha*-gene or at least a part thereof or an introduction of at least one mutation into the *ldha*-gene;

C) a deletion of the *pflD*-gene or at least a part thereof, a deletion of a regulatory element of the *pflD*-gene or at least a part thereof or an introduction of at least one mutation into the *pflD*-gene;

D) a deletion of the *pflA*-gene or at least a part thereof, a deletion of a regulatory element of the *pflA*-gene or at least a part thereof or an introduction of at least one mutation into the *pflA*-gene;

E) a deletion of the *ldha*-gene or at least a part thereof, a deletion of a regulatory element of the *ldha*-gene or at least a part thereof or an introduction of at least one mutation into the *ldha*-gene
and
a deletion of the *pflD*-gene or at least a part thereof, a deletion of a regulatory element of the *pflD*-gene or at least a part thereof or an introduction of at least one mutation into the *pflD*-gene;
or

F) a deletion of the *ldha*-gene or at least a part thereof, a deletion of a regulatory element of the *ldha*-gene or at least a part thereof or an introduction of at least one mutation into the *ldha*-gene
and
a deletion of the *pflA*-gene or at least a part thereof, a deletion of a regulatory element of the *pflA*-gene or at least a part thereof or an introduction of at least one mutation into the *pflA*-gene.

Particular preferred embodiments of the genetically modified microorganisms used in the process according to the present invention are:

- modified bacterial cells of the genus *Basfia,* preferably of the species *Basfia succiniciproducens,* most preferably of the species *Basfia succiniciproducens* strain DD1, in which the *udhA*-gene is overexpressed (preferably by integration of the *udhA*-gene into the chromosome of the cell under control of the *pckA*-promoter or the *gapA*-promoter);

- modified bacterial cells of the genus *Basfia,* preferably of the species *Basfia succiniciproducens,* most preferably of the species *Basfia succiniciproducens* strain DD1, in which the *udhA*-gene is overexpressed (preferably by integration of the *udhA*-gene into the chromosome of the cell under control of the *pckA*-promoter or the *gapA*-promoter), and in which in addition to these genetic modifications the activity of the lactate dehydrogenase is reduced, preferably by a modification of the *ldhA*-gene, in particular by a deletion of the *ldha*-gene having the nucleic acid sequence according to **SEQ ID NO: 7** and encoding for LdhA having the amino acid sequence according to **SEQ ID NO: 8**;

- modified bacterial cells of the genus *Basfia,* preferably of the species *Basfia succiniciproducens,* most preferably of the species *Basfia succiniciproducens* strain DD1, in which the *udhA*-gene is overexpressed (preferably by integration of the *udhA*-gene into the chromosome of the cell under control of the *pckA*-promoter or the *gapA*-promoter), and in which in addition to these genetic modifications the activity of the pyruvate formate lyase is reduced, preferably by a modification of the *pflA*-gene or the *pflD*-gene, in particular by a modification of the *pflA*-gene having the nucleic acid sequence according to **SEQ ID NO: 9** and encoding for PflA having the amino acid sequence according to **SEQ ID NO: 10** or by a modification of the *pflD*-gene having the nucleic acid sequence according to **SEQ ID NO: 11** and encoding for PflD having the amino acid sequence according to **SEQ ID NO: 12**;

- modified bacterial cells of the genus *Basfia,* preferably of the species *Basfia succiniciproducens,* most preferably of the species *Basfia succiniciproducens* strain DD1, in which the *udhA*-gene is overexpressed (preferably by integration of the into the chromosome of the cell under control of the *pckA*-promoter or the *gapA*-promoter), and in which in addition to these genetic modifications the activity of the lactate dehydrogenase and the pyruvate formate lyase is reduced, preferably by a modification of the *ldha*-gene and the *pflA*-gene, in particular by a modification of the *ldha*-gene having the nucleic acid sequence according to **SEQ ID NO: 7** and encoding for LdhA having the amino acid sequence according to **SEQ ID NO: 8** or by a modification of the *pflA*-gene having the nucleic acid sequence according to **SEQ ID NO: 9** and encoding for PflA having the amino acid sequence according to **SEQ ID**

**NO: 10**, or a modification of the *ldha*-gene and the *pflD*-gene, in particular by a modification of the *ldha*-gene having the nucleic acid sequence according to **SEQ ID NO: 7** and encoding for LdhA having the amino acid sequence according to **SEQ ID NO: 8** or by a modification of the *pflD*-gene having the nucleic acid sequence according to **SEQ ID NO: 11** and encoding for PflD having the amino acid sequence according to **SEQ ID NO: 12.**

In process step I) the genetically modified microorganism according to the present invention is cultured in a culture medium comprising sucrose as the assimilable carbon source to allow the modified microorganism to produce the organic compound, thereby obtaining a fermentation broth comprising the organic compound. Preferred organic compounds that can be produced by the process according to the present invention comprise carboxylic acids such as formic acid, lactic acid, propionic acid, 2-hydroxypropionic acid, 3-hydroxypropionic acid, 3-hydroxybutyric acid, acrylic acid, pyruvic acid or salts of these carboxylic acids, dicarboxylic acids such as ma-lonic acid, succinic acid, malic acid, tartaric acid, glutaric acid, itaconic acid, adipic acid or salts thereof, tricarboxylic acids such as citric acid or salts thereof, alcohols such as methanol or ethanol, amino acids such as L-asparagine, L-aspartic acid, L-arginine, L-isoleucine, L-glycine, L-glutamine, L-glutamic acid, L-cysteine, L-serine, L-tyrosine, L-tryptophan, L-threonine, L-valine, L-histidine, L-proline, L-methionine, L-lysine, L-leucine, etc..

According to a preferred embodiment of the process according to the present invention the organic compound is succinic acid. The term *"succinic acid*", as used in the context of the present invention, has to be understood in its broadest sense and also encompasses salts thereof (i. e. succinate), as for example alkali metal salts, like $Na^+$ and $K^+$-salts, or earth alkali salts, like $Mg^{2+}$ and $Ca^{2+}$-salts, or ammonium salts or anhydrides of succinic acid.

The genetically modified microorganism according to the present invention is, preferably, incubated in the culture medium at a temperature in the range of about 10 to 60°C or 20 to 50°C or 30 to 45°C at a pH of 5.0 to 9.0 or 5.5 to 8.0 or 6.0 to 7.0.

Preferably, the organic compound, especially succinic acid, is produced under anaerobic conditions. Anaerobic conditions may be established by means of conventional techniques, as for example by degassing the constituents of the reaction medium and maintaining anaerobic conditions by introducing carbon dioxide or nitrogen or mixtures thereof and optionally hydrogen at a flow rate of, for example, 0.1 to 1 or 0.2 to 0.5 vvm. Aerobic conditions may be established by means of conventional techniques, as for example by introducing air or oxygen at a flow rate of, for example, 0.1 to 1 or 0.2 to 0.5 vvm. If appropriate, a slight over pressure of 0.1 to 1.5 bar may be applied in the process.

The assimilable carbon source is preferably selected from sucrose, maltose, maltotriose, maltotetraose, maltopentaose, maltohexaose, maltoheptaose, D-fructose, D-glucose, D-xylose, L-arabinose, D-galactose, D-mannose, glycerol and mixtures thereof or compositions containing at least one of said compounds, or is selected from decomposition products of starch, cellulose, hemicellulose and/or lignocellulose. Preferably, the assimilable carbon source comprises D-glucose, maltose, sucrose, glycerol or a mixture of at least two of these compounds, wherein mixtures of glycerol and D-glucose, glycerol and sucrose, glycerol and D-xylose, glycerol and maltose and D-glucose and fructose are particularly preferred.

The initial concentration of the assimilable carbon source is preferably adjusted to a value in a range of 5 to 100 g/l, preferably 5 to 75 g/l and more preferably 5 to 50 g/l and may be maintained in said range during cultivation. The pH of the reaction medium may be controlled by addition of suitable bases as for example, gaseous ammonia, $NH_4HCO_3$, $(NH_4)2CO_3$, NaOH, $Na_2CO_3$, $NaHCO_3$, KOH, $K_2CO_3$, $KHCO_3$, $Mg(OH)_2$, $MgCO_3$, $Mg(HCO_3)_2$, $Ca(OH)_2$, $CaCO_3$, $Ca(HCO_3)_2$, CaO, $CH_6N_2O_2$, $C_2H_7N$ and/or mixtures thereof. These alkaline neutralization agents are especially required if the organic compounds that are formed in the course of the fermentation process are carboxylic acids or dicarboxylic acids. In the case of succinic acid as the organic compound, $Mg(OH)_2$ is a particular preferred base.

The fermentation step I) according to the present invention can, for example, be performed in stirred fermenters, bubble columns and loop reactors. A comprehensive overview of the possible method types including stirrer types and geometric designs can be found in Chmiel: "Bioprozesstechnik: Einführung in die Bioverfahrenstechnik", Volume 1. In the process according to the present invention, typical variants available are the following variants known to those skilled in the art or explained, for example, in Chmiel, Hammes and Bailey: "Biochemical Engineering*",* such as batch, fed-batch, repeated fed-batch or else continuous fermentation with and without recycling of the biomass. Depending on the production strain, sparging with air, oxygen, carbon dioxide, hydrogen, nitrogen or appropriate gas mixtures may be effected in order to achieve good yield (YP/S).

Particularly preferred conditions for producing the organic acid, especially succinic acid, in process step I) are:

| | |
|---|---|
| Temperature: | 30 to 45°C |
| pH: | 5.5 to 7.0 |
| Supplied gas: | $CO_2$ |

It is furthermore preferred in process step I) that the assimilable carbon source is converted to the organic compound, preferably to succinic acid, with a carbon yield YP/S of at least 0.5 g/g up to about 1.18 g/g; as for example a carbon yield YP/S of at least 0,6 g/g, of at least 0.7 g/g, of at least 0.75 g/g, of at least 0.8 g/g, of at least 0.85 g/g, of at least 0.9 g/g, of at least 0.95 g/g, of at least 1.0 g/g, of at least 1.05 g/g or of at least 1.1 g/g (organic compound/carbon, preferably succinic acid/carbon).

It is furthermore preferred in process step I) that the assimilable carbon source is converted to the organic compound, preferably to succinic acid, with a specific productivity yield of at least 0.6 g g $DCW^{-1}h^{-1}$ organic compound, preferably succinic acid, or of at least of at least 0.65 g g $DCW^{-1}h^{-1}$, of at least 0.7 g g $DCW^{-1}h^{-1}$, of at least 0.75 g g $DCW^{-1}h^{-1}$ or of at least 0.77 g g $DCW^{-1}h^{-1}$ organic compound, preferably succinic acid.

It is furthermore preferred in process step I) that the assimilable carbon source is converted to the organic compound, preferably to succinic acid, with a space time yield for the organic compound, preferably for succinic acid, of at least 2.2 g/(L×h) or of at least 2.5 g/(L×h), at least 2.75 g/(L×h), at least 3 g/(L×h), at least 3.25 g/(L×h), at least 3.5 g/(L×h), at least 3.7 g/(L×h), at least 4.0 g/(L×h) at least 4.5 g/(L×h) or at least 5.0 g/(L×h) of the organic compound, preferably succinic acid. According to another preferred embodiment of the process according to the present invention in process step I) the genetically modified microorganism is converting at least 20 g/L, more preferably at least 25 g/l and even more preferably at least 30 g/l sucrose to at least 20 g/l, more preferably to at least 25 g/l and even more preferably at least 30 g/l of the organic compound, preferaby succinic acid.

The different yield parameters as described herein ("*carbon yield*" or "*YP/S*"; *"specific productivity yield*"; or *"space-time-yield (STY)")* are well known in the art and are determined as described for example by Song and Lee, 2006. "Carbon yield" and "YP/S" (each expressed in mass of organic compound produced/mass of assimilable carbon source consumed) are herein used as synonyms. The specific productivity yield describes the amount of a product, like succinic acid, that is produced per h and L fermentation broth per g of dry biomass. The amount of dry cell weight stated as "*DCW*" describes the quantity of biologically active microorganism in a biochemical reaction. The value is given as g product per g DCW per h (i.e. g g $DCW^{-1}h^{-1}$). The space-time-yield (STY) is defined as the ratio of the total amount of organic compound formed in the fermentation process to the volume of the culture, regarded over the entire time of cultivation. The space-time yield is also known as the *"volumetric productivity*".

In process step II) the organic compound, preferably succinic acid, is recovered from the fermentation broth obtained in process step I).

Ususally, the recovery process comprises the step of separating the genetically modified microrganims from the fermentation broth as the so called "biomass". Processes for removing the biomass are known to those skilled in the art, and comprise filtration, sedimentation, flotation or combinations thereof. Consequently, the biomass can be removed, for example, with centrifuges, separators, decanters, filters or in a flotation apparatus. For maximum recovery of the product of value, washing of the biomass is often advisable, for example in the form of a diafiltration. The selection of the method is dependent upon the biomass content in the fermentation broth and the properties of the biomass, and also the interaction of the biomass with the organic compound (e. the product of value). In one embodiment, the fermentation broth can be sterilized or pasteurized. In a further embodiment, the fermentation broth is concentrated. Depending on the requirement, this concentration can be done batch wise or continuously. The pressure and temperature range should be selected such that firstly no product damage occurs, and secondly minimal use of apparatus and energy is necessary. The skillful selection of pressure and temperature levels for a multistage evaporation in particular enables saving of energy.

The recovery process may further comprise additional purification steps in which the organic compound, preferably succinic acid, is further purified. If, however, the organic compound is converted into a secondary organic product by chemical reactions as described below, a further purification of the organic compound is, depending on the kind of reaction and the reaction conditions, not necessarily required. For the purification of the organic compound obtained in process step II), preferably for the purification of succinic acid, methods known to the person skilled in the art can be used, as for example crystallization, filtration, electrodialysis and chromatography. In the case of succinic acid as the organic compound, for example, succinic acid may be isolated by precipitating it as a calcium succinate product by using calcium hydroxide, -oxide, - carbonate or hydrogen carbonate for neutralization and filtration of the precipitate. The succinic acid is recovered from the precipitated calcium succinate by acidification with sulfuric acid followed by filtration to remove the calcium sulfate (gypsum) which precipitates. The resulting solution may be further purified by means of ion exchange chromatography in order to remove undesired residual ions. Alternatively, if magnesium hydroxide, magnesium carbonate or mixtures thereof have been used to neutralize the fermentation broth, the fermentation broth obtained in process step I) may be acidified to transform the magnesium succinate contained in the medium into the acid form (i. e. succinic acid), which subsequently can be crystallized by cooling down the acidified medium. Examples of further suitable purification processes are disclosed in EP-A-1 005 562, WO-A-2008/010373, WO-A-2011/082378, WO-A-2011/043443, WO-A-2005/030973, WO-A-2011/123268 and WO-A-2011/064151 and EP-A-2 360 137.

According to a preferred embodiment of the process according to the present invention the process further comprises the process step:

l) conversion of the organic compound contained in the fermentation broth obtained in process step I) or conversion of the recovered organic compound obtained in process step II) into a secondary organic product being different from the organic compound by at least one chemical reaction.

In case of succinic acid as the organic compound preferred secondary organic products are selected from the group consisting of succinic acid esters and polymers thereof, tetrahydrofuran (THF), 1,4-butanediol (BDO), gamma-butyrolactone (GBL) and pyrrolidones.

According to a preferred embodiment for the production of THF, BDO and/or GBL this process comprises:

b1) either the direct catalytic hydrogenation of the succinic acid obtained in process steps I) or II) to THF and/or BDO and/or GBL or

b2) the chemical esterification of succinic acid and/or succinic acid salts obtained in process steps I) or II) into its corresponding di-lower alkyl ester and subsequent catalytic hydrogenation of said ester to THF and/or BDO and/or GBL.

According to a preferred embodiment for the production of pyrrolidones this process comprises:

b) the chemical conversion of succinic acid ammonium salts obtained in process steps I) or II) to pyrrolidones in a manner known per se.

For details of preparing these compounds reference is made to US-A-2010/0159543 and WO-A-2010/092155.

A contribution to solving the problems mentioned at the outset is furthermore provided by a genetically modified microorganism that comprises

A) at least one genetic modification that leads to an increased activity of the enzyme encoded by the *udhA*-gene, compared to the original microorganism that has not been genetically modified,

wherein the original microorganism belongs to the family *Pasteurellaceae.*

Preferred embodiments of the genetically modified microorganisms according to the present invention are those embodiments that have been described above as preferred genetically modified microorganisms to be used in the process according to the present invention.

A contribution to solving the problems mentioned at the outset is furthermore provided by the use of the genetically modified microorganism according to the present invention for the fermentative production of organic compounds, preferably succinic acid, from assimilable carbon sources such as glycerol, glucose, sucrose, xylose, lactose, fructose or maltose. Preferred organic compounds, preferred assimilable carbon sources and preferred conditions for the fermentative production of organic compounds are those compounds, carbon sources and conditions that have already been described in connection with process step I) of the process according to the present invention.

The invention is now explained in more detail with the aid of non-limiting figures and examples.

Figure 1 shows a schematic map of plasmid pSacB-P*gapA-udhA* (**SEQ ID NO: 13**).

Figure 2 shows a schematic map of plasmid pSacB-P*pckA-udhA* (**SEQ ID NO: 14**).

## EXAMPLES

Example 1: Generation of constructs

The plasmids pSacB-P*pckA-udhA* (fig. 1) and pSacB-P*gapA-udhA* (fig. 2) for chromosomal integration and overexpression of the *E. coli udhA*-gene in *Basfia succiniciproducens* strain DD1 *ΔldhAΔpflD* were constructed based on the vector pSacB which was already described in WO 2015/118111 A1. The 5'- and 3'- flanking regions (approx. 1500 bp each) for integration of the *udhA* expression cassette in the *Basfia succiniciproducens* bioB locus as well as the *E. coli udhA* gene and the Basfia *pckA*- or *gapA* promoter sequences were amplified via PCR and introduced into vector pSacB using the CPEC (Circular Polymerase Extension Cloning) method, which enables seamless assembly of multiple DNA fragments based on overlapping sequences (Quan and Tian 2009, PLoS ONE 4: e6441).

Example 2: Generation of modified *Basfia succiniciproducens* strains

Vector and strain construction were conducted by standard techniques as described previously (Becker et al., Biotechnology and Bioengineering, Vol. 110 (2013), pages 3013-3023). All mutants constructed are listed in Table 1:

Table 1: Nomenclature of the DD1-mutants referred to in the examples Specific primer sequences used for strain construction and validation are given in Table 2:

| strain |
|---|
| DD1 Δ*ldhA*ΔpflD |
| DD1 Δ*ldhA*ΔpflD P*gapA*::*udhA* |
| DD1 Δ*ldhA*ΔpflD P*pckA*::*udhA* |

Table 2: primer sequenced used in the examples

| Primer | Sequence 5' → 3' |
|---|---|
| $PR_{udhA}1$ | gagtacgttattcagcgccg (**SEQ ID NO: 13**) |
| $PR_{udhA}2$ | acgaatcactacgccactgt (**SEQ ID NO: 14**) |
| $PR_{udhA}3$ | gcgccatgtgctgatttacg (**SEQ ID NO: 15**) |
| $PR_{udhA}4$ | cgatattcggcacatcaggcac (**SEQ ID NO: 16**) |

Mutant strains DD1 Δ*ldM*ΔpflD were prepared as described in WO 2015/118111 A1.

For overexpression of NAD(P)$^+$-transhydrogenase (*udhA*), mutant strain DD1 Δ*ldA*ΔpflD was transformed as described in WO 2015/118111 A1 with the plasmids pSacB-P*pckA*-*udhA* (fog. 1) and pSacB-P*gapA*-*udhA* (fig. 2), respectively, and "Campbelled in" to yield a "Campbell in" strain. Transformation and integration into the genome was confirmed by PCR analysis yielding bands for the integration event of the plasmids into the genome of *Basfia succiniciproducens.* Primer pairs $PR_{udhA}1$/$PR_{udhA}2$ as well as $PR_{udhA}3$/$PR_{udhA}4$ were used to identify 5'- or 3' plasmid integration events. The "Campbelled in" strains were then "Campbelled out" using agar plates containing sucrose as a counter selection medium, selecting for the loss (of function) of the *sacB* gene. Therefore, the "Campbelled in" strains were incubated in 25-35 ml of non-selective BHI medium containing no chloramphenicol (BHI medium Becton Dickinson, containing the following additions MOPS: 9.4 g/l, $Mg(OH)_2$: 0.625 g/l, BIS-TRIS: 5.8 g/l, $NaHCO_3$: 1.8 g/l) at 37°C, 220 rpm over night. The overnight culture was then streaked onto freshly prepared BHI containing 10% sucrose plates (no chloramphenicol) and incubated overnight at 37°C ("first sucrose transfer"). Single colony obtained from first transfer were again streaked onto freshly prepared BHI containing 10% sucrose plates and incubated overnight at 37°C ("second sucrose transfer"). This procedure was repeated until a minimal completion of five transfers ("third, forth, fifth sucrose transfer") onto sucrose. The term "first to fifth sucrose transfer" refers to the transfer of a strain after chromosomal integration of a vector containing a *sacB*-levan-sucrase gene onto sucrose and growth mediun containing agar plates for the purpose of selecting strains with the loss of the *sacB* gene and the surrounding plasmid sequences. After the last transfer, colonies were streaked onto BHI plates containing 10% sucrose (no chloramphenicol) to obtain single colonies. Single colonies were then streaked simultaneously on BHI plates i) without sucrose and without chloramphenicol and ii) without sucrose and with 5 mg/L chloramphenicol. The "Campbelled out" strains containing the additional copy of the *udhA* gene under the *pckA* or *gapA* promoter sequence were identified by chloramphenicol sensitivity. Colonies, which did not grow on plates with chloramphenicol, were tested in PCR analyses with the use of primer pairs $PR_{udhA}1$/$PR_{udhA}2$ as well as $PR_{udhA}3$/$PR_{udhA}4$. Clones showing the proper PCR amplicons were re-streaked onto BHI plates and single colonies were obtained and tested again in the above described PCR reaction to ensure single colonies and exclude mixed cultures. The described procedure led to the generation of strains DD1 Δ*ldA*ΔpflD P*gapA*::*udhA and* DD1 Δ*ldA*ΔpflD P*pckA*::*udhA.*

Example 3: Cultivation of DD1 Δ*ldA*ΔpflD, DD1 Δ*ldA*ΔpflD P*gapA*::*udhA* and DD1 Δ*ldhA*ΔpflD P*pckA*::*udhA*

The productivity of the DD1 Δ*ldA*ΔpflD-strain was compared with the productivity of the DD1 Δ/*dhA*ΔpflD P*gapA*::*udhA* strain and the DD1 Δ*ldA*ΔpflD P*pckA*::*udhA* strain in the presence of glucose as a carbon source. Productivity was analyzed utilizing media and incubation conditions described below.

1. Medium preparation

For physiological studies and for succinate production, pre-cultivation of *B. succiniciproducens* was conducted in complex CGM medium, while main cultivation was done in LSM3 minimal medium with 50 g/l gucose. Carbon dioxide was applied to the culture bottles (serum flasks) at 0.8 bar overpressure. Composition and preparation of the CGM and the LSM3 cultivation medium is as described in the following Tables 3, 4, 5 and 6.

Table 3: Composition of CGM cultivation medium

| Compound | Concentration [g/l] |
|---|---|
| Yeast extract | 12.5 |
| Succinic acid | 2.5 |
| $(NH_4)_2SO_4$ | 0.5 |
| $KH_2PO_4$ | 1.0 |
| $MgCO_3$ | 50.0 |
| $Na_2CO_3$ | 2.0 |
| Glucose | 52 |

Table 4: Composition of the trace element solution 5

| Compound | Final concentration |
|---|---|
| Citric acid | 10 g/l |
| $ZnSO_4$ x 7 $H_2O$ | 1851 mg/l |
| $CaSO_4$ x 2 $H_2O$ | 10 mg/l |
| $FeSO_4$ x 7 $H_2O$ | 2040 mg/l |
| $CaCl_2$ x 2 $H_2O$ | 12460 mg/l |
| $MnCl_2$ x 4 $H_2O$ | 1200 mg/l |
| $Na_2MoO_4$ x 2 $H_2O$ | 38 mg/l |
| $CuCl_2$ x 2 $H_2O$ | 188 mg/l |
| $NiCl_2$ x 6 $H_2O$ | 32 mg/l |
| $CoCl_2$ x 6 $H_2O$ | 101 mg/l |

Table 5: Composition of the vitamin solution 9

| Compound | Final concentration |
|---|---|
| Thiamin HCl (B1) | 1 g/l |
| Nicotinic acid (B3) | 1 g/l |
| Riboflavin (B2) | 20 mg/l |
| Biotin (B7) | 50 mg/l |
| Pantothenic acid (B5) | 1 g/l |
| Pyridoxine (B6) | 1 g/l |
| Cyanocobalamin (B12) | 50 mg/l |
| Lipoic acid | 5 mg/l |

Table 6: Composition of the LSM3 medium with glucose

| **Compound** | **Volume/Mass** | **Stock conc.** | **Final conc.** |
|---|---|---|---|
| Medium 1 | | | |
| $MgCO_3$ | 2.5 g | 100% | 50 g/l |
| $H_2O$ | 38.45 ml | - | - |
| Medium 2 | | | |
| Succinic acid | 2.5 ml | 50 g/l | 2.5 g/l |
| Glucose | 4 ml | 650 g/l | 52 g/l |
| $(NH_4)_2SO_4$ | 0.5 ml | 500 g/l | 5 g/l |
| Betain | 0.5 ml | 23 g/l | 0.23 g/l |
| $KH_2PO_4$ | 0.5 ml | 100 g/l | 1 g/l |
| $Na_2CO_3$ | 0.5 ml | 200 g/l | 2 g/l |
| Vitamin solution 9 (conc. 100x) | 0.5 ml | 4 g/l | 0.04 g/l |
| Trace element solution 5 | 0.05 ml | 21 g/l | 0.02 g/l |

2. Cultivations

For growing the pre-culture, *Basfia succiniciproducens* colonies from a freshly grown BHI agar plate (incubated overnight at 37°C under anaerobic conditions) was used to inoculate a 100 ml serum bottle with gas tight butyl rubber stopper containing 50 ml of the CGM liquid medium described in Table 3 with $CO_2$ atmosphere. The bottles were incubated at 37°C and 170 rpm (shaking diameter: 2.5 cm). For growing the main culture, 2.5 ml of the bacterial culture in the CGM medium (after 10 hours of incubation) was used to inoculate a 100 ml serum bottle with gas tight butyl rubber stopper containing 50 ml of the LSM3 glucose containing medium as described in Table 6 with a $CO_2$ atmosphere at 0.8 bar overpressure.

3. Analytics

Succinic acid, lactic acid, formic acid, acetic acid, pyruvic acid, propionic acid and ethynaol were analyzed by HPLC. Cell growth was monitored by measuring the absorbance at 600 nm using a spectrophotometer.

4. Results

Table 7 shows the formation of succinic acid from glucose as the sole carbon source when the cells are cultured in a batch process:

| | DD1 Δ*ldhA*ΔpflD | DD1 Δ*ldhA*ΔpflD P*gapA*:: *udhA* | DD1 Δ*ldhA*ΔpflD P*pckA*:: *udhA* |
|---|---|---|---|
| substrate | glucose | glucose | glucose |
| tc [h]a | 24 | 24 | 24 |
| $\Delta c_{substrate}$ [g/l][b] | 49.95 | 51.56 | 53.01 |
| $\Delta c_{SA}$ [g/l][b] (succinic aicid) | 31.45 | 38.15 | 37.64 |
| $\Delta c_{LA}$ [g/l][c] (lactic acid) | 0.22 | 0.29 | 0.31 |
| $\Delta$ $c_{FA}$ [g/l][c,h] (formic acid) | 0.00 | 0.00 | 0.00 |
| Δ CAA [g/l][c,h] (acetic acid) | 2.89 | 2.70 | 2.21 |
| $\Delta$ $c_{PA}$ [g/l][c,h] (pyruvic acid) | 1.87 | 1.48 | 1.77 |

(continued)

| | DD1 Δ*ldhA*ΔpflD | DD1 Δ*ldhA*ΔpflD P*gapA*:: *udhA* | DD1 Δ*ldhA*ΔpflD P*pckA*:: *udhA* |
|---|---|---|---|
| Δ $c_P$ [g/l][c,h] (propionic acid) | 0.00 | 0.00 | 0.00 |
| Δ $c_E$ [g/l][c] (ethanol) | 0.00 | 0.00 | 0.00 |
| SA Yield (SA/S) [g/g][g] | 0.63 | 0.74 | 0.71 |

[a] cultivation time
[b] consumption of substrate
[c] formation of succinic acid, lactic acid, formic acid, acetic acid, pyruvic acid propionic acid and ethanol
[g] succinic acid yield (ration of succinic acid per consumed substrate)
[h] Detection limits for acetic acid, lactic acid, and formic acid were found to be lower than 0.01 g/L in the given HPLC method

**SEQUENCES**

**SEQ ID NO: 1** (nucleotide sequence of 16 S rDNA of strain DD1)

tttgatcctggctcagattgaacgctggcggcaggcttaacacatgcaagtcgaacggtagcgggaggaaagcttgctttctttgccga
cgagtggcggacgggtgagtaatgcttggggatctggcttatggagggggataacgacgggaaactgtcgctaataccgcgtaatat
cttcggattaaagggtgggactttcgggccacccgccataagatgagcccaagtgggattaggtagttggtggggtaaaggcctacc
aagccgacgatctctagctggtctgagaggatgaccagccacactggaactgagacacggtccagactcctacgggaggcagca
gtggggaatattgcacaatgggggaaccctgatgcagccatgccgcgtgaatgaagaaggccttcgggttgtaaagttctttcggtg
acgaggaaggtgtttgttttaataggacaagcaattgacgttaatcacagaagaagcaccggctaactccgtgccagcagccgcggt
aatacggagggtgcgagcgttaatcggaataactgggcgtaaagggcatgcaggcggacttttaagtgagatgtgaaagccccgg
gcttaacctgggaattgcatttcagactgggagtctagagtactttagggaggggtagaattccacgtgtagcggtgaaatgcgtagag
atgtggaggaataccgaaggcgaaggcagccccttgggaagatactgacgctcatatgcgaaagcgtggggagcaaacaggatt
agataccctggtagtccacgcggtaaacgctgtcgatttggggattgggctttaggcctggtgctcgtagctaacgtgataaatcgacc
gcctggggagtacggccgcaaggttaaaactcaaatgaattgacgggggcccgcacaagcggtggagcatgtggtttaattcgatg
caacgcgaagaaccttacctactcttgacatccagagaatcctgtagagatacgggagtgccttcgggagctctgagacaggtgctg
catggctgtcgtcagctcgtgttgtgaaatgttgggttaagtcccgcaacgagcgcaacccttatcctttgttgccagcatgtaaagatgg
gaactcaaaggagactgccggtgacaaaccggaggaaggtggggatgacgtcaagtcatcatggcccttacgagtagggctaca
cacgtgctacaatggtgcatacagagggcggcgataccgcgaggtagagcgaatctcagaaagtgcatcgtagtccggattggagt
ctgcaactcgactccatgaagtcggaatcgctagtaatcgcaaatcagaatgttgcggtgaatacgttcccgggccttgtacacaccg
cccgtcacaccatgggagtgggttgtaccagaagtagatagcttaaccttcggggggggcgtttaccacggtatgattcatgactggg
gtgaagtcgtaacaaggtaaccgtaggggaacctgcgg

**SEQ ID NO: 2** (nucleotide sequence of 23 S rDNA of strain DD1)

agtaataacgaacgacacaggtataagaatacttgaggttgtatggttaagtgactaagcgtacaaggtggatgccttggcaatcaga ggcgaagaaggacgtgctaatctgcgaaaagcttgggtgagttgataagaagcgtctaacccaagatatccgaatggggcaaccc agtagatgaagaatctactatcaataaccgaatccataggttattgaggcaaaccgggagaactgaaacatctaagtaccccgagg aaaagaaatcaaccgagattacgtcagtagcggcgagcgaaagcgtaagagccggcaagtgatagcatgaggattagaggaat cggctgggaagccgggcggcacagggtgatagccccgtacttgaaaatcattgtgtggtactgagcttgcgagaagtagggcggga cacgagaaatcctgtttgaagaagggggaccatcctccaaggctaaatactcctgattgaccgatagtgaaccagtactgtgaagg aaaggcgaaaagaaccccggtgaggggagtgaaatagaacctgaaaccttgtacgtacaagcagtgggagcccgcgagggtga ctgcgtaccttttgtataatgggtcagcgacttatattatgtagcgaggttaaccgaataggggagccgaagggaaaccgagtcttaact gggcgtcgagttgcatgatatagacccgaaacccggtgatctagccatgggcaggttgaaggttgggtaacactaactggaggacc gaaccgactaatgttgaaaaattagcggatgacctgtggctgggggtgaaaggccaatcaaaccgggagatagctggttctccccg aaatctatttaggtagagccttatgtgaataccttcgggggtagagcactgtttcggctagggggccatcccggcttaccaacccgatgc aaactgcgaataccgaagagtaatgcataggagacacacggcgggtgctaacgttcgtcgtggagagggaaacaacccagacc gccagctaaggtcccaaagtttatattaagtgggaaacgaagtgggaaggcttagacagctaggatgttggcttagaagcagccatc atttaaagaaagcgtaatagctcactagtcgagtcggcctgcgcggaagatgtaacggggctcaaatatagcaccgaagctgcggc atcaggcgtaagcctgttgggtaggggagcgtcgtgtaagcggaagaaggtggttcgagagggctgctggacgtatcacgagtgcg aatgctgacataagtaacgataaaacgggtgaaaaacccgttcgccggaagaccaagggttcctgtccaacgttaatcggggcag ggtgagtcggcccctaaggcgaggctgaagagcgtagtcgatgggaaacgggttaatattcccgtacttgttataattgcgatgtggg gacggagtaggttaggttatcgacctgttggaaaaggtcgtttaagttggtaggtggagcgtttaggcaaatccggacgcttatcaaca

ccgagagatgatgacgaggcgctaaggtgccgaagtaaccgataccacacttccaggaaaagccactaagcgtcagattataata aaccgtactataaaccgacacaggtggtcaggtagagaatactcaggcgcttgagagaactcgggtgaaggaactaggcaaaata gcaccgtaacttcgggagaaggtgcgccggcgtagattgtagaggtataccttgaaggttgaaccggtcgaagtgacccgctggct gcaactgtttattaaaaacacagcactctgcaaacacgaaagtggacgtatagggtgtgatgcctgcccggtgctggaaggttaattg atggcgttatcgcaagagaagcgcctgatcgaagccccagtaaacggcggccgtaactataacggtcctaaggtagcgaaattcctt gtcgggtaagttccgacctgcacgaatggcataatgatggccaggctgtctccacccgagactcagtgaaattgaaatcgccgtgaa gatgcggtgtacccgcggctagacggaaagaccccgtgaacctttactatagcttgacactgaaccttgaattttgatgtgtaggatag gtgggaggctttgaagcggtaacgccagttatcgtggagccatccttgaaataccaccctttaacgtttgatgttctaacgaagtgcccg gaacgggtactcggacagtgtctggtgggtagtttgactggggcggtctcctcccaaagagtaacggaggagcacgaaggtttgcta atgacggtcggacatcgtcaggttagtgcaatggtataagcaagcttaactgcgagacggacaagtcgagcaggtgcgaaagcag gtcatagtgatccggtggttctgaatggaagggccatcgctcaacggataaaaggtactccggggataacaggctgataccgccca agagttcatatcgacggcggtgtttggcacctcgatgtcggctcatcacatcctggggctgaagtaggtcccaagggtatggctgttcgc catttaaagtggtacgcgagctgggtttaaaacgtcgtgagacagtttggtccctatctgccgtgggcgttggagaattgagaggggct gctcctagtacgagaggaccggagtggacgcatcactggtgttccggttgtgtcgccagacgcattgccgggtagctacatgcggaa gagataagtgctgaaagcatctaagcacgaaacttgcctcgagatgagttctcccagtatttaatactgtaagggttgttggagacgac gacgtagataggccgggtgtgtaagcgttgcgagacgttgagctaaccggtactaattgcccgagaggcttagccatacaacgctca agtgtttttggtagtgaaagttattacggaataagtaagtagtcagggaatcggct

**SEQ ID NO: 3** (nucleotide sequence of from *E. coli*)

atgccacattcctacgattacgatgccatagtaataggttccggccccggcggcgaaggcgctgcaatgggcctggttaagcaaggt
gcgcgcgtcgcagttatcgagcgttatcaaaatgttggcggcggttgcacccactggggcaccatcccgtcgaaagctctccgtcacg
ccgtcagccgcattatagaattcaatcaaaacccactttacagcgaccattcccgactgctccgctcttcttttgccgatatccttaaccat
gccgataacgtgattaatcaacaaacgcgcatgcgtcagggattttacgaacgtaatcactgtgaaatattgcagggaaacgctcgct
ttgttgacgagcatacgttggcgctggattgcccggacggcagcgttgaaacactaaccgctgaaaaatttgttattgcctgcggctctc
gtccatatcatccaacagatgttgatttcacccatccacgcatttacgacagcgactcaattcttagcatgcaccacgaaccgcgccat
gtgctgatttacggtgctggagtgatcggctgtgaatatgcgtcgatcttccgcggtatggatgtaaaagtggatctgatcaacacccgc
gatcgcctgctggcatttctcgatcaagagatgtcagattctctctcctatcacttctggaacagtggcgtagtgattcgtcacaacgaag
agtacgagaagatcgaaggctgtgacgatggtgtgatcatgcatttgaagtcgggtaaaaaactgaaagctgactgcctgctctatgc
caacggtcgcaccggtaataccgattcgctggcgttacagaacattgggctggaaactgacagtcgcggacagctgaaggtcaaca
gcatgtatcagaccgcacagccgcacgtttacgcggtgggcgacgtgattggttatccgagcctggcatcagcggcctatgaccagg
ggcgcattgccgcgcaggcgctggtaaaaggcgaagccaccgcgcatctgattgaagatatccctaccggcatttacaccatcccg
gaaatcagctctgtgggcaaaaccgaacagcagctgaccgcgatgaaagtgccatatgaagtgggccgcgcccagtttaaacatct
ggcacgcgcacaaatcgtcggcatgaacgtgggcacgctgaaaattttgttccatcgggaaacaaaagagattctgggtattcactg
ctttggcgagcgcgctgccgaaattattcatatcggtcaggcgattatggaacagaaaggtggcggcaacactattgagtacttcgtca
acaccacctttaactacccgaccatggcggaagcctatcgggtagctgcgttaaacggtttaaaccgcctgttttaa

**SEQ ID NO: 4** (amino acid sequence of NAD(P)$^+$-transhydrogenase from *E. coli*)

MPHSYDYDAIVIGSGPGGEGAAMGLVKQGARVAVIERYQNVGGGCTHWGTIPSKALRHAVSRI
IEFNQNPLYSDHSRLLRSSFADILNHADNVINQQTRMRQGFYERNHCEILQGNARFVDEHTLAL
DCPDGSVETLTAEKFVIACGSRPYHPTDVDFTHPRIYDSDSILSMHHEPRHVLIYGAGVIGCEYA
SIFRGMDVKVDLINTRDRLLAFLDQEMSDSLSYHFWNSGVVIRHNEEYEKIEGCDDGVIMHLKS
GKKLKADCLLYANGRTGNTDSLALQNIGLETDSRGQLKVNSMYQTAQPHVYAVGDVIGYPSLA

SAAYDQGRIAAQALVKGEATAHLIEDIPTGIYTIPEISSVGKTEQQLTAMKVPYEVGRAQFKHLA
RAQIVGMNVGTLKILFHRETKEILGIHCFGERAAEIIHIGQAIMEQKGGGNTIEYFVNTTFNYPTM

**SEQ ID NO: 5** (nucleotide sequence of *pckA-promoter* from strain DD1)

ttattttcatatattttccaataatttgatctagataacatttttaggtatcttttgtcctataatgcgaaacattagtttttattatttttaatttatttt
atgaggtgatgt

**SEQ ID NO: 6** (nucleotide sequence of *gapA-promoter* from strain DD1)

accatttgtcaatactcattttgttaaatttcatagcgttaaatattgacatggatcacaaaaaagttatctgttatagtttagcgcatagtcta
gtttcgaatataaaagaaatcgtgctataatttcgaaatgacggatagttgaaataataatattttgatgaccc

**SEQ ID NO: 7** (nucleotide sequence of *ldha*-gene from strain DD1)

ttgacaaaatcagtatgtttaaataaggagctaactatgaaagttgccgtttacagtactaaaaattatgatcgcaaacatctggatttgg
cgaataaaaaatttaattttgagcttcatttctttgattttttacttgatgaacaaaccgcgaaaatggcggagggcgccgatgccgtctgta
ttttcgtcaatgatgatgcgagccgcccggtgttaacaaagttggcgcaaatcggagtgaaaattatcgctttacgttgtgccggttttaat
aatgtggatttggaggcggcaaaagagctgggattaaaagtcgtacgggtgcctgcgtattcgccggaagccgttgccgagcatgcg
atcggattaatgctgactttaaaccgccgtatccataaggcttatcagcgtacccgcgatgcgaatttttctctggaaggattggtcggtttt
aatatgttcggcaaaaccgccggagtgattggtacgggaaaaatcggcttggcggctattcgcattttaaaaggcttcggtatggacgtt
ctggcgtttgatccttttaaaaatccggcggcggaagcgttgggcgcaaaatatgtcggtttagacgagctttatgcaaaatcccatgtta
tcactttgcattgcccggctacggcggataattatcatttattaaatgaagcggcttttaataaaatgcgcgacggtgtaatgattattaata
ccagccgcggcgttttaattgacagccgggcggcaatcgaagcgttaaaacggcagaaaatcggcgctctcggtatggatgtttatg
aaaatgaacgggatttgtttttcgaggataaatctaacgatgttattacggatgatgtattccgtcgcctttcttcctgtcataatgtgctttttac
cggtcatcaggcgtttttaacggaagaagcgctgaataatatcgccgatgtgactttatcgaatattcaggcggtttccaaaaatgcaac
gtgcgaaaatagcgttgaaggctaa

**SEQ ID NO: 8** (amino acid sequence of LdhA from strain DD1)

MTKSVCLNKELTMKVAVYSTKNYDRKHLDLANKKFNFELHFFDFLLDEQTAKMAEGADAVCIFV
NDDASRPVLTKLAQIGVKIIALRCAGFNNVDLEAAKELGLKVVRVPAYSPEAVAEHAIGLMLTLN
RRIHKAYQRTRDANFSLEGLVGFNMFGKTAGVIGTGKIGLAAIRILKGFGMDVLAFDPFKNPAAE
ALGAKYVGLDELYAKSHVITLHCPATADNYHLLNEAAFNKMRDGVMIINTSRGVLIDSRAAIEAL
KRQKIGALGMDVYENERDLFFEDKSNDVITDDVFRRLSSCHNVLFTGHQAFLTEEALNNIADVT
LSNIQAVSKNATCENSVEG

**SEQ ID NO: 9** (nucleotide sequence of pflA-gene from strain DD1)

atgtcggttttaggacgaattcattcatttgaaacctgcgggacagttgacgggccgggaatccgctttattttatttttacaaggctgcttaa
tgcgttgtaaatactgccataatagagacacctgggatttgcacggcggtaaagaaatttccgttgaagaattaatgaaagaagtggtg
acctatcgccattttatgaacgcctcgggcggcggagttaccgcttccggcggtgaagctattttacaggcggaatttgtacgggactgg
ttcagagcctgccataaagaaggaattaatacttgcttggataccaacggtttcgtccgtcatcatgatcatattattgatgaattgattgat
gacacggatcttgtgttgcttgacctgaaagaaatgaatgaacgggttcacgaaagcctgattggcgtgccgaataaaagagtgctcg
aattcgcaaaatatttagcggatcgaaatcagcgtacctggatccgccatgttgtagtgccgggttatacagatagtgacgaagatttgc
acatgctggggaatttcattaaagatatgaagaatatcgaaaaagtggaattattaccttatcaccgtctaggcgcccataaatgggaa
gtactcggcgataaatacgagcttgaagatgtaaaaccgccgacaaaagaattaatggagcatgttaaggggttgcttgcaggctac

gggcttaatgtgacatattag

**SEQ ID NO: 10** (amino acid sequence of PflA from strain DD1)

MSVLGRIHSFETCGTVDGPGIRFILFLQGCLMRCKYCHNRDTWDLHGGKEISVEELMKEVVTY
RHFMNASGGGVTASGGEAILQAEFVRDWFRACHKEGINTCLDTNGFVRHHDHIIDELIDDTDLV
LLDLKEMNERVHESLIGVPNKRVLEFAKYLADRNQRTWIRHVVVPGYTDSDEDLHMLGNFIKD
MKNIEKVELLPYHRLGAHKWEVLGDKYELEDVKPPTKELMEHVKGLLAGYGLNVTY

**SEQ ID NO: 11** (nucleotide sequence of *pflD*-gene from strain DD1)

atggctgaattaacagaagctcaaaaaaaagcatgggaaggattcgttcccggtgaatggcaaaacggcgtaaatttacgtgacttt
atccaaaaaaactatactccgtatgaaggtgacgaatcattcttagctgatgcgactcctgcaaccagcgagttgtggaacagcgtga
tggaaggcatcaaaatcgaaaacaaaactcacgcacctttagatttcgacgaacatactccgtcaactatcacttctcacaagcctgg
ttatatcaataaagatttagaaaaaatcgttggtcttcaaacagacgctccgttaaaacgtgcaattatgccgtacggcggtatcaaaat
gatcaaaggttcttgcgaagtttacggtcgtaaattagatccgcaagtagaatttattttcaccgaatatcgtaaaacccataaccaagg
cgtattcgacgtttatacgccggatattttacgctgccgtaaatcaggcgtgttaaccggtttaccggatgcttacggtcgtggtcgtattatc
ggtgactaccgtcgtttagcggtatacggtattgattacctgatgaaagataaaaaagcccaattcgattcattacaaccgcgtttggaa
gcgggcgaagacattcaggcaactatccaattacgtgaagaaattgccgaacaacaccgcgctttaggcaaaatcaaagaaatgg
cggcatcttacggttacgacatttccggccctgcgacaaacgcacaggaagcaatccaatggacatattttgcttatctggcagcggtt
aaatcacaaaacggtgcggcaatgtcattcggtcgtacgtctacattcttagatatctatatcgaacgtgacttaaaacgcggtttaatca
ctgaacaacaggcgcaggaattaatggaccacttagtaatgaaattacgtatggttcgtttcttacgtacgccggaatacgatcaattatt
ctcaggcgacccgatgtgggcaaccgaaactatcgccggtatgggcttagacggtcgtccgttggtaactaaaaacagcttccgcgt
attacatactttatacactatgggtacttctccggaaccaaacttaactattctttggtccgaacaattacctgaagcgttcaaacgtttctgt
gcgaaagtatctattgatacttcctccgtacaatacgaaaatgatgacttaatgcgtcctgacttcaacaacgatgactatgcaatcgcat
gctgcgtatcaccgatggtcgtaggtaaacaaatgcaattcttcggtgcgcgcgcaaacttagctaaaactatgttatacgcaattaac
ggcggtatcgatgagaaaaatggtatgcaagtcggtcctaaaactgcgccgattacagacgaagtattgaatttcgataccgtaatcg
aacgtatggacagtttcatggactggttggcgactcaatatgtaaccgcattgaacatcatccacttcatgcacgataaatatgcatatg
aagcggcattgatggcgttccacgatcgcgacgtattccgtacaatggcttgcggtatcgcgggtctttccgtggctgcggactcattatc
cgcaatcaaatatgcgaaagttaaaccgattcgcggcgacatcaaagataaagacggtaatgtcgtggcctcgaatgttgctatcga
cttcgaaattgaaggcgaatatccgcaattcggtaacaatgatccgcgtgttgatgatttagcggtagacttagttgaacgtttcatgaaa
aaagttcaaaaacacaaaacttaccgcaacgcaactccgacacaatctatcctgactatcacttctaacgtggtatacggtaagaaa
accggtaatactccggacggtcgtcgagcaggcgcgccattcggaccgggtgcaaacccaatgcacggtcgtgaccaaaaaggt
gcggttgcttcacttacttctgtggctaaacttccgttcgcttacgcgaaagacggtatttcatataccttctctatcgtaccgaacgcattag
gtaaagatgacgaagcgcaaaaacgcaaccttgccggtttaatggacggttatttccatcatgaagcgacagtggaaggcggtcaa
cacttgaatgttaacgttcttaaccgtgaaatgttgttagacgcgatggaaaatccggaaaaatacccgcaattaaccattcgtgtttcag
gttacgcggttcgtttcaactcattaactaaagagcaacaacaagacgtcatcactcgtacgtttacacaatcaatgtaa

**SEQ ID NO: 12** (amino acid of PflD from strain DD1)

MAELTEAQKKAWEGFVPGEWQNGVNLRDFIQKNYTPYEGDESFLADATPATSELWNSVMEGI
KIENKTHAPLDFDEHTPSTITSHKPGYINKDLEKIVGLQTDAPLKRAIMPYGGIKMIKGSCEVYGR
KLDPQVEFIFTEYRKTHNQGVFDVYTPDILRCRKSGVLTGLPDAYGRGRIIGDYRRLAVYGIDYL
MKDKKAQFDSLQPRLEAGEDIQATIQLREEIAEQHRALGKIKEMAASYGYDISGPATNAQEAIQ
WTYFAYLAAVKSQNGAAMSFGRTSTFLDIYIERDLKRGLITEQQAQELMDHLVMKLRMVRFLRT

PEYDQLFSGDPMWATETIAGMGLDGRPLVTKNSFRVLHTLYTMGTSPEPNLTILWSEQLPEAF
KRFCAKVSIDTSSVQYENDDLMRPDFNNDDYAIACCVSPMVVGKQMQFFGARANLAKTMLYAI
NGGIDEKNGMQVGPKTAPITDEVLNFDTVIERMDSFMDWLATQYVTALNIIHFMHDKYAYEAAL
MAFHDRDVFRTMACGIAGLSVAADSLSAIKYAKVKPIRGDIKDKDGNVVASNVAIDFEIEGEYPQ
FGNNDPRVDDLAVDLVERFMKKVQKHKTYRNATPTQSILTITSNVVYGKKTGNTPDGRRAGAP
FGPGANPMHGRDQKGAVASLTSVAKLPFAYAKDGISYTFSIVPNALGKDDEAQKRNLAGLMDG
YFHHEATVEGGQHLNVNVLNREMLLDAMENPEKYPQLTIRVSGYAVRFNSLTKEQQQDVITRT
FTQSM

**SEQ ID NO: 13** (nucleotide sequence of pSacB-P*gapA-udhA*)

gtcttgcctgctttatcagtaacaaacccgcgcgatttacttttcgacctcattctattagactctcgtttggattgcaactggtctattttcctcttt
tgtttgatagaaaatcataaaaggatttgcagactacgggcctaaagaactaaaaaatctatctgtttcttttcattctctgtatttttatagttt
ctgttgcatgggcataaagttgcctttttaatcacaattcagaaaatatcataatatctcatttcactaaataatagtgaacggcaggtatat
gtgatgggttaaaaaggatcggcggccgctcgatttaaatcccgctttccgcctgtttatcgctgttaatgtcaatgggattgttgttgctatc
acgccgtctggtatggctgcattttacgcaaattgccaatgttattatgaacttaggtatgttgatattagccgtgcccggtttaattttagctgt
cgggttgtttttactgttgcaaaaaatggaatttggtaccgcgcatttatttgccgtcatggtgatgtgtaacgctttttcggcaatgccgtttgt
gatcagaattcttgcgcccggcatgaataacaatatgcagtattacgaaaaattatgtcaatcgctgggtattcggggctggcaacgtttt
cgcctgatcgagcaacacaaactcgcccagccgataaaatacgcttttgccttagcctgtaccttatctttgggtgattttacggcgattgc
gctattcggcagccagcaattttcttctttgccttatttgctctatcaacagttgggcagttatcgaggagatcaagccgccgttacggcatt
ggttttacttttaatgtgcctgcttatctttatactggtcgaaggcgctaaaaattcagataaaaacgagaacgcgaatgataaaacttga
acgggtatattttaattataaaactatgccgatgaattttaatattcatattaaaccgcaggagcgggtagcgattatcggtgccagcgga
gcggggaaaagcacattattaaatcttatcgccggttttgaacgggcggatgacggcgaaatttggttgaacggcgtaaaccatactta
tacggaaccttatgaacgccccgtatccatgttgtttcaggaaaataacttgtttacccatttaaccgttgaacaaaatatcgctttagggct
aaagcccgatttaaaacttagcgccgccgaacaatcactggttcgacaaaccgcaagtgcggtcggtttaagccgatttttagataga
aaacccaccgcgctttccggcgggcagaagcagcgggtggcattagcgcgttgtttgttacgcaataagccgattttattgctggacga
acctttttccgcattggatccggcattaagagcggaaatgctcgatttgctttcgcaactttgtaatgaaaaaaactcactttgctgatcgt
cactcatcaaccgagcgaactacaaggacgaatagacagaattttgaccgttgaaaacgggcattttgcaaagaataatgatctaaa
atagctcggaaacggtagctatgtattagaaaacttcaaatagcaagagataaaataccttgcaatgcgttactaacataaggatttga
gatgaaaacaacattacaactttattcgaatacacctcatcctcaggtggaatattggtctgtttgtaaggtggaggcgcttttcgaaacg
ccgtttttagaattagtgcatcgtgcggctttagttcatcgtgaaaattttaatccgcaagttattcaactctcgaccttaatgtcaatcaaaa
ccggcggttgtccggaagattgcggttattgtccccaatccgctcgctaaaccatttgtcaatactcattttgttaaatttcatagcgttaaat
attgacatggatcacaaaaaagttatctgttatagtttagcgcatagtctagtttcgaatataaaagaaatcgtgctataatttcgaaatga
cggatagttgaaataataatattttgatgacccatgccacattcctacgattacgatgccatagtaataggttccggccccggcggcgaa
ggcgctgcaatgggcctggttaagcaaggtgcgcgcgtcgcagttatcgagcgttatcaaaatgttggcggcggttgcacccactggg
gcaccatcccgtcgaaagctctccgtcacgccgtcagccgcattatagaattcaatcaaaacccactttacagcgaccattcccgact
gctccgctcttcttttgccgatatccttaaccatgccgataacgtgattaatcaacaaacgcgcatgcgtcagggattttacgaacgtaat
cactgtgaaatattgcagggaaacgctcgctttgttgacgagcatacgttggcgctggattgcccggacggcagcgttgaaacactaa
ccgctgaaaaatttgttattgcctgcggctctcgtccatatcatccaacagatgttgatttcacccatccacgcatttacgacagcgactca
attcttagcatgcaccacgaaccgcgccatgtgctgatttacggtgctggagtgatcggctgtgaatatgcgtcgatcttccgcggtatgg
atgtaaaagtggatctgatcaacacccgcgatcgcctgctggcatttctcgatcaagagatgtcagattctctctcctatcacttctggaa
cagtggcgtagtgattcgtcacaacgaagagtacgagaagatcgaaggctgtgacgatggtgtgatcatgcatttgaagtcgggtaa
aaaactgaaagctgactgcctgctctatgccaacggtcgcaccggtaataccgattcgctggcgttacagaacattggctggaaact

gacagtcgcggacagctgaaggtcaacagcatgtatcagaccgcacagccgcacgtttacgcggtgggcgacgtgattggttatcc
gagcctggcatcagcggcctatgaccaggggcgcattgccgcgcaggcgctggtaaaaggcgaagccaccgcgcatctgattga
agatatccctaccggcatttacaccatcccggaaatcagctctgtgggcaaaaccgaacagcagctgaccgcgatgaaagtgccat
atgaagtgggccgcgcccagtttaaacatctggcacgcgcacaaatcgtcggcatgaacgtgggcacgctgaaaattttgttccatcg
ggaaacaaaagagattctgggtattcactgctttggcgagcgcgctgccgaaattattcatatcggtcaggcgattatggaacagaaa
ggtggcggcaacactattgagtacttcgtcaacaccacctttaactacccgaccatggcggaagcctatcgggtagctgcgttaaacg
gtttaaaccgcctgttttaacgccgtttatatttttaatagcgtacctaatcgggtacgcttttttttatgcggaaaatccatatttttctaccgcact
ttttctttaaagatttatacttaagtctgtttgattcaatttatttggaggttttatgcaacacattcaactggctcccgatttaacattcagtcgctt
aattcagggattctggcggttaaaaagctggcggaaatcgccgcaggaagcaagaatcactatgccaagaagttatgtgcgtttatcc
gccgggcgacaaggcatgtcggaggaaatgcaggcgatgtgttttatggcgggtgccaactctatttttatggcgataaattactggtt
accggcaatgcggaagaagattgcgatagactgctaatggaaaaattggatttagaaccggaaaccactgaaaaccgttacctttcg
caaaatagttaaagcctgttaataggagagaacatgtttaaagttcagcggatttatgatttgaaccgatggaaaatgattgcgcggta
tttgtggatcggctttatccgcggggcgtaaataaagaaaaatttgcccattgcctatggctaaaggatgtgtgcccgagtcatgagttac
gccggttttatcatgaaaatccgcaggaaaattatggagaatttgtgctccgttatcaactggaattaggtaacgaattgccgcaaaaa
ggattgatgatgcttaaacgtctggagaaagaacatccgcaggttattttgctgactgccgttaaagatgtgcgacacagccatatccc
ggtcttattgaaagcgttagccgctattgtagaatttctataaagcaaaaagtgcggtaaaatttttttcaaaatttccaccgcactttagattt
tgcaataagattgtaatggtgggctggcaagcccaccctaccgatctgacctgcggcaagtcatatcctggtcttattgaaagcgttagc
cgatttttcataaaaaaagccacacaaatgtgtggcttaaacagttcctaataataatcaaacagctaagtaaatctgcaattatagtttg
tcggcgttttgttttaagtattttgcaacgccttccgggctgtctttcataccttctttgccttttttcccattgagcagggcaaacttcaccgtgttg
ctcgtggaattgtaaagcgtcaaccatacgtaacatttcgtcgatattacggcctaacggtaaatcgttcacaacttggtgacgaactac
gccgtttgcatcgattaagaaagaggcacgtaatgccacgccggcttccggatgttcaatgccgtatgcttttgcgatttcgtgtttagtgtc
tgccgctaaagcatattgaacttgaccgataccgccttgatctaccgcggtattacgccatgcgttgtgagtaaattgagagtcgattga
aacgccaaccacttcaacaccgcgtttttgaattcttcataacgatgatcaaatgcgattaattcagacgggcaaacgaaagtaaagt
ctaacggatagaagaaaattaccgccggtttaccagcaatatgttgtttgaaattgaagttatcaacaatttcgccgttgcctaaaacgg
ctgctgatgtaaagtccggtgcttgacgtgtaactaataccatgtttaatctcctaatagaataatcgaatgaatattttgcgtactttcgtaa
atcatcgcaaaatcaacaatttctattctaagaaaaagaagtcaatttgaatagttgattttatctatcaaattaattatcgttatctaacatta
aaaaacaggaagtattatgtcaaacaaatattcacttgcaacgacgctggttcacgccggtcgtagcaaacgcgttagccagggtag
tgtaaatccggtggtgcaacgcgcatcttcattggttttcgatagtatcgcagataaacgacaagcaaccgttaaccactccataggcc
gctttcctggctttgcttccagatgtatgctctcctccggagagtaccgtgactttattttcggcacaaatacaggggtcgatggataaatac
ggcgatagtttcctgacggatgatccgtatgtaccggcggaagacaagctgcaaacctgtcagatggagattgatttaatggcggatgt
gctgagagcaccgccccgtgaatccgcagaactgatccgctatgtgtttgcggatgattggccggaataaataaagccgggcttaata
cagattaagcccgtatagggtattattactgaataccaaacagcttacggaggacggaatgttacccattgagacaaccagactgcct
tctgattattaatatttttcactattaatcagaaggaataaccatgaattttacccggattgacctgaatacctggaatcgcagggaacactt
tgccctttatcgtcagcagattaaatgcggattcagcctgaccaccaaactcgatattaccgctttgcgtaccgcactggcggagacag
gttataagttttatccgctgatgatttacctgatctcccgggctgttaatcagtttccggagttccggatggcactgaaagacaatgaactta
tttactgggaccagtcagacccggtctttactgtctttcataaagaaaccgaaacattctctgcactgtcctgccgttattttccggatctcag
tgagtttatggcaggttataatgcggtaacggcagaatatcagcatgataccagattgtttccgcagggaaatttaccggagaatcacct
gaatatatcatcattaccgtgggtgagttttgacgggatttaacctgaacatcaccggaaatgatgattattttgccccggtttttacgatgg
caaagtttcagcaggaaggtgaccgcgtattattacctgtttctgtacaggttcatcatgcagtctgtgatggctttcatgcagcacggttta
ttaatacacttcagctgatgtgtgataacatactgaaataaattaattaattctgtatttaagccaccgtatccggcaggaatggtggcttttt
ttttatattttaaccgtaatctgtaatttcgtttcagactggttcaggatcactgtacgataatgcccccgcagtttggtaatacccttaataaa
aaagaaacagcaaagactgacagcaataagagctcgcttggactcctgttgatagatccagtaatgacctcagaactccatctggat

ttgttcagaacgctcggttgccgccgggcgttttttattggtgagaatccaagcactagcggcgcgccggccggcccggtgtgaaatac
cgcacagatgcgtaaggagaaaataccgcatcaggcgctcttccgcttcctcgctcactgactcgctgcgctcggtcgttcggctgcg
gcgagcggtatcagctcactcaaaggcggtaatacggttatccacagaatcaggggataacgcaggaaagaacatgtgagcaaa
aggccagcaaaaggccaggaaccgtaaaaaggccgcgttgctggcgtttttccataggctccgcccccctgacgagcatcacaaa
aatcgacgctcaagtcagaggtggcgaaacccgacaggactataaagataccaggcgtttccccctggaagctccctcgtgcgctct
cctgttccgaccctgccgcttaccggatacctgtccgcctttctcccttcgggaagcgtggcgctttctcatagctcacgctgtaggtatctc
agttcggtgtaggtcgttcgctccaagctgggctgtgtgcacgaaccccccgttcagcccgaccgctgcgccttatccggtaactatcgt
cttgagtccaacccggtaagacacgacttatcgccactggcagcagccactggtaacaggattagcagagcgaggtatgtaggcgg
tgctacagagttcttgaagtggtggcctaactacggctacactagaaggacagtatttggtatctgcgctctgctgaagccagttaccttc
ggaaaaagagttggtagctcttgatccggcaaacaaaccaccgctggtagcggtggtttttttgtttgcaagcagcagattacgcgcag
aaaaaaaggatctcaagaagatcctttgatcttttctacggggtctgacgctcagtggaacgaaaactcacgttaagggattttggtcat
gagattatcaaaaaggatcttcacctagatccttttaaaggccggccgcggccgccatcggcattttcttttgcgtttttatttgttaactgtta
attgtccttgttcaaggatgctgtctttgacaacagatgttttcttgcctttgatgttcagcaggaagctcggcgcaaacgttgattgtttgtctg
cgtagaatcctctgtttgtcatatagcttgtaatcacgacattgtttcctttcgcttgaggtacagcgaagtgtgagtaagtaaaggttacatc
gttaggatcaagatccatttttaacacaaggccagttttgttcagcggcttgtatgggccagttaaagaattagaaacataaccaagcat
gtaaatatcgttagacgtaatgccgtcaatcgtcatttttgatccgcgggagtcagtgaacaggtaccatttgccgttcattttaaagacgtt
cgcgcgttcaatttcatctgttactgtgttagatgcaatcagcggtttcatcactttttttcagtgtgtaatcatcgtttagctcaatcataccgag
agcgccgtttgctaactcagccgtgcgttttttatcgctttgcagaagtttttgactttcttgacggaagaatgatgtgcttttgccatagtatgc
tttgttaaataaagattcttcgccttggtagccatcttcagttccagtgtttgcttcaaatactaagtatttgtggcctttatcttctacgtagtgag
gatctctcagcgtatggttgtcgcctgagctgtagttgccttcatcgatgaactgctgtacattttgatacgtttttccgtcaccgtcaaagatt
gatttataatcctctacaccgttgatgttcaaagagctgtctgatgctgatacgttaacttgtgcagttgtcagtgtttgtttgccgtaatgtttac
cggagaaatcagtgtagaataaacggatttttccgtcagatgtaaatgtggctgaacctgaccattcttgtgtttggtcttttaggatagaat
catttgcatcgaatttgtcgctgtctttaaagacgcggccagcgtttttccagctgtcaatagaagtttcgccgactttttgatagaacatgta
aatcgatgtgtcatccgcatttttaggatctccggctaatgcaaagacgatgtggtagccgtgatagtttgcgacagtgccgtcagcgtttt
gtaatggccagctgtcccaaacgtccaggccttttgcagaagagatatttttaattgtggacgaatcaaattcagaaacttgatatttttcat
ttttttgctgttcagggatttgcagcatatcatggcgtgtaatatgggaaatgccgtatgtttccttatatggcttttggttcgtttctttcgcaaac
gcttgagttgcgcctcctgccagcagtgcggtagtaaaggttaatactgttgcttgttttgcaaactttttgatgttcatcgttcatgtctcctttt
tatgtactgtgttagcggtctgcttcttccagccctcctgtttgaagatggcaagttagttacgcacaataaaaaagacctaaaatatgta
aggggtgacgccaaagtatacactttgccctttacacattttag

**SEQ ID NO: 14** (nucleotide sequence of pSacB-P*pckA*-*udhA*

gtcttgcctgctttatcagtaacaaacccgcgcgatttacttttcgacctcattctattagactctcgtttggattgcaactggtctattttcctcttt
tgtttgatagaaaatcataaaaggatttgcagactacgggcctaaagaactaaaaaatctatctgtttcttttcattctctgtatttttttatagttt
ctgttgcatgggcataaagttgcctttttaatcacaattcagaaaatatcataatatctcatttcactaaataatagtgaacggcaggtatat
gtgatgggttaaaaaggatcggcggccgctcgatttaaatcccgctttccgcctgtttatcgctgttaatgtcaatgggattgttgttgctatc
acgccgtctggtatggctgcattttacgcaaattgccaatgttattatgaacttaggtatgttgatattagccgtgcccggtttaattttagctgt
cgggttgtttttactgttgcaaaaaaatggaatttggtaccgcgcatttatttgccgtcatggtgatgtgtaacgctttttcggcaatgccgtttgt
gatcagaattcttgcgcccggcatgaataacaatatgcagtattacgaaaaattatgtcaatcgctgggtattcggggctggcaacgtttt
cgcctgatcgagcaacacaaactcgcccagccgataaaatacgcttttgccttagcctgtaccttatctttgggtgattttacggcgattgc
gctattcggcagccagcaattttcttctttgccttatttgctctatcaacagttgggcagttatcgaggagatcaagccgccgttacggcatt
ggttttacttttaatgtgcctgcttatctttatactggtcgaaggcgctaaaaattcagataaaaacgagaacgcgaatgataaaacttga
acgggtatattttaattataaaactatgccgatgaattttaatattcatattaaaccgcaggagcgggtagcgattatcggtgccagcgga

gcggggaaaagcacattattaaatcttatcgccggttttgaacgggcggatgacggcgaaatttggttgaacggcgtaaaccatactta
tacggaaccttatgaacgccccgtatccatgttgtttcaggaaaataacttgtttacccatttaaccgttgaacaaaatatcgctttagggct
aaagcccgatttaaaacttagcgccgccgaacaatcactggttcgacaaaccgcaagtgcggtcggtttaagccgatttttagataga
aaacccaccgcgctttccggcgggcagaagcagcgggtggcattagcgcgttgtttgttacgcaataagccgattttattgctggacga
acctttttccgcattggatccggcattaagagcggaaatgctcgatttgctttcgcaactttgtaatgaaaaaaaactcactttgctgatcgt
cactcatcaaccgagcgaactacaaggacgaatagacagaattttgaccgttgaaaacgggcattttgcaaagaataatgatctaaa
atagctcggaaacggtagctatgtattagaaaacttcaaatagcaagagataaaataccttgcaatgcgttactaacataaggatttga
gatgaaaacaacattacaactttattcgaatacacctcatcctcaggtggaatattggtctgtttgtaaggtggaggcgcttttcgaaacg
ccgtttttagaattagtgcatcgtgcggctttagttcatcgtgaaaattttaatccgcaagttattcaactctcgaccttaatgtcaatcaaaa
ccggcggttgtccggaagattgcggttattgtccccaatccgctcgctaattattttcatatattttccaataatttgatctagataacatttttta
ggtatcttttgtcctataatgcgaaacattagtttttattatttttaatttattttatgaggtgatgtatgccacattcctacgattacgatgccata
gtaataggttccggccccggcggcgaaggcgctgcaatgggcctggttaagcaaggtgcgcgcgtcgcagttatcgagcgttatcaa
aatgttggcggcggttgcacccactggggcaccatcccgtcgaaagctctccgtcacgccgtcagccgcattatagaattcaatcaaa
acccactttacagcgaccattcccgactgctccgctcttcttttgccgatatccttaaccatgccgataacgtgattaatcaacaaacgcg
catgcgtcagggattttacgaacgtaatcactgtgaaatattgcagggaaacgctcgctttgttgacgagcatacgttggcgctggattg
cccggacggcagcgttgaaacactaaccgctgaaaaatttgttattgcctgcggctctcgtccatatcatccaacagatgttgatttcacc
catccacgcatttacgacagcgactcaattcttagcatgcaccacgaaccgcgccatgtgctgatttacggtgctggagtgatcggctgt
gaatatgcgtcgatcttccgcggtatggatgtaaaagtggatctgatcaacacccgcgatcgcctgctggcatttctcgatcaagagatg
tcagattctctctcctatcacttctggaacagtggcgtagtgattcgtcacaacgaagagtacgagaagatcgaaggctgtgacgatgg
tgtgatcatgcatttgaagtcgggtaaaaaactgaaagctgactgcctgctctatgccaacggtcgcaccggtaataccgattcgctgg
cgttacagaacattgggctggaaactgacagtcgcggacagctgaaggtcaacagcatgtatcagaccgcacagccgcacgtttac
gcggtgggcgacgtgattggttatccgagcctggcatcagcggcctatgaccaggggcgcattgccgcgcaggcgctggtaaaagg
cgaagccaccgcgcatctgattgaagatatccctaccggcatttacaccatcccggaaatcagctctgtgggcaaaaccgaacagc
agctgaccgcgatgaaagtgccatatgaagtgggccgcgcccagtttaaacatctggcacgcgcacaaatcgtcggcatgaacgtg
ggcacgctgaaaattttgttccatcgggaaacaaaagagattctgggtattcactgctttggcgagcgcgctgccgaaattattcatatc
ggtcaggcgattatggaacagaaaggtggcggcaacactattgagtacttcgtcaacaccacctttaactacccgaccatggcggaa
gcctatcgggtagctgcgttaaacggtttaaaccgcctgttttaacgccgtttatatttttaatagcgtacctaatcgggtacgctttttttatgc
ggaaaatccatatttttctaccgcactttttctttaaagatttatacttaagtctgtttgattcaatttatttggaggttttatgcaacacattcaact
ggctcccgatttaacattcagtcgcttaattcagggattctggcggttaaaaagctggcggaaatcgccgcaggaagcaagaatcact
atgccaagaagttatgtgcgtttatccgccgggcgacaaggcatgtcggaggaaatgcaggcgatgtgttttatggcgggtgccaact
ctatttttatggcgataaattactggttaccggcaatgcggaagaagattgcgatagactgctaatggaaaaattggatttagaaccgg
aaaccactgaaaaccgttacctttcgcaaaatagttaaagcctgttaataggagagaacatgtttaaagttcagcggatttatgattttga
accgatggaaaatgattgcgcggtatttgtggatcggctttatccgcggggcgtaaataaagaaaaatttgcccattgcctatggctaaa
ggatgtgtgcccgagtcatgagttacgccggttttatcatgaaaatccgcaggaaaattatggagaatttgtgctccgttatcaactggaa
ttaggtaacgaattgccgcaaaaaggattgatgatgcttaaacgtctggagaaagaacatccgcaggttattttgctgactgccgttaa
agatgtgcgacacagccatatcccggtcttattgaaagcgttagccgctattgtagaatttctataaagcaaaaagtgcggtaaaattttt
caaaatttccaccgcactttagattttgcaataagattgtaatggtgggctggcaagcccaccctaccgatctgacctgcggcaagtcat
atcctggtcttattgaaagcgttagccgatttttcataaaaaaagccacacaaatgtgtggcttaaacagttcctaataataatcaaacag
ctaagtaaatctgcaattatagtttgtcggcgttttgttttaagtattttgcaacgccttccgggctgtctttcataccttctttgcctttttcccattg
agcagggcaaacttcaccgtgttgctcgtggaattgtaaagcgtcaaccatacgtaacatttcgtcgatattacggcctaacggtaaatc
gttcacaacttggtgacgaactacgccgtttgcatcgattaagaaagaggcacgtaatgccacgccggcttccggatgttcaatgccgt
atgcttttgcgatttcgtgtttagtgtctgccgctaaagcatattgaacttgaccgataccgccttgatctaccgcggtattacgccatgcgtt

gtgagtaaattgagagtcgattgaaacgccaaccacttcaacaccgcgtttttgaattcttcataacgatgatcaaatgcgattaattca
gacgggcaaacgaaagtaaagtctaacggatagaagaaaattaccgccggtttaccagcaatatgttgtttgaaattgaagttatcaa
caatttcgccgttgcctaaaacggctgctgatgtaaagtccggtgcttgacgtgtaactaataccatgtttaatctcctaatagaataatcg
aatgaatattttgcgtactttcgtaaatcatcgcaaaatcaacaatttctattctaagaaaaagaagtcaatttgaatagttgattttatctatc
aaattaattatcgttatctaacattaaaaaacaggaagtattatgtcaaacaaatattcacttgcaacgacgctggttcacgccggtcgta
gcaaacgcgttagccagggtagtgtaaatccggtggtgcaacgcgcatcttcattggttttcgatagtatcgcagataaacgacaagc
aaccgttaaccactccataggccgctttcctggctttgcttccagatgtatgctctcctccggagagtaccgtgactttattttcggcacaaa
tacaggggtcgatggataaatacggcgatagtttcctgacggatgatccgtatgtaccggcggaagacaagctgcaaacctgtcaga
tggagattgatttaatggcggatgtgctgagagcaccgccccgtgaatccgcagaactgatccgctatgtgtttgcggatgattggccg
gaataaataaagccgggcttaatacagattaagcccgtatagggtattattactgaataccaaacagcttacggaggacggaatgtta
cccattgagacaaccagactgccttctgattattaatatttttcactattaatcagaaggaataaccatgaattttacccggattgacctga
atacctggaatcgcagggaacactttgccctttatcgtcagcagattaaatgcggattcagcctgaccaccaaactcgatattaccgctt
tgcgtaccgcactggcggagacaggttataagttttatccgctgatgatttacctgatctcccgggctgttaatcagtttccggagttccgg
atggcactgaaagacaatgaacttatttactgggaccagtcagacccggtctttactgtctttcataaagaaaccgaaacattctctgca
ctgtcctgccgttattttccggatctcagtgagtttatggcaggttataatgcggtaacggcagaatatcagcatgataccagattgtttccg
cagggaaatttaccggagaatcacctgaatatatcatcattaccgtgggtgagttttgacgggatttaacctgaacatcaccggaaatg
atgattattttgccccggtttttacgatggcaaagtttcagcaggaaggtgaccgcgtattattacctgtttctgtacaggttcatcatgcagt
ctgtgatggctttcatgcagcacggtttattaatacacttcagctgatgtgtgataacatactgaaataaattaattaattctgtatttaagcc
accgtatccggcaggaatggtggcttttttttatattttaaccgtaatctgtaatttcgtttcagactggttcaggatcactgtacgataatgcc
cccgcagtttggtaatacccttaataaaaagaaacagcaaagactgacagcaataagagctcgcttggactcctgttgatagatcc
agtaatgacctcagaactccatctggatttgttcagaacgctcggttgccgccgggcgttttttattggtgagaatccaagcactagcggc
gcgccggccggcccggtgtgaaataccgcacagatgcgtaaggagaaaataccgcatcaggcgctcttccgcttcctcgctcactg
actcgctgcgctcggtcgttcggctgcggcgagcggtatcagctcactcaaaggcggtaatacggttatccacagaatcaggggata
acgcaggaaagaacatgtgagcaaaaggccagcaaaaggccaggaaccgtaaaaaggccgcgttgctggcgtttttccataggc
tccgcccccctgacgagcatcacaaaaatcgacgctcaagtcagaggtggcgaaacccgacaggactataaagataccaggcgtt
tccccctggaagctccctcgtgcgctctcctgttccgaccctgccgcttaccggatacctgtccgcctttctcccttcgggaagcgtggcg
ctttctcatagctcacgctgtaggtatctcagttcggtgtaggtcgttcgctccaagctgggctgtgtgcacgaaccccccgttcagcccg
accgctgcgccttatccggtaactatcgtcttgagtccaacccggtaagacacgacttatcgccactggcagcagccactggtaacag
gattagcagagcgaggtatgtaggcggtgctacagagttcttgaagtggtggcctaactacggctacactagaaggacagtatttggt
atctgcgctctgctgaagccagttaccttcggaaaaagagttggtagctcttgatccggcaaacaaaccaccgctggtagcggtggtttt
tttgtttgcaagcagcagattacgcgcagaaaaaaaggatctcaagaagatcctttgatcttttctacggggtctgacgctcagtggaac
gaaaactcacgttaagggattttggtcatgagattatcaaaaaggatcttcacctagatccttttaaaggccggccgcggccgccatcg
gcattttcttttgcgtttttatttgttaactgttaattgtccttgttcaaggatgctgtctttgacaacagatgttttcttgcctttgatgttcagcagga
agctcggcgcaaacgttgattgtttgtctgcgtagaatcctctgtttgtcatatagcttgtaatcacgacattgtttcctttcgcttgaggtaca
gcgaagtgtgagtaagtaaaggttacatcgttaggatcaagatccatttttaacacaaggccagttttgttcagcggcttgtatgggcca
gttaaagaattagaaacataaccaagcatgtaaatatcgttagacgtaatgccgtcaatcgtcatttttgatccgcgggagtcagtgaac
aggtaccatttgccgttcattttaaagacgttcgcgcgttcaatttcatctgttactgtgttagatgcaatcagcggtttcatcactttttttcagtg
tgtaatcatcgtttagctcaatcataccgagagcgccgtttgctaactcagccgtgcgttttttatcgctttgcagaagtttttgactttcttgac
ggaagaatgatgtgcttttgccatagtatgctttgttaaataaagattcttcgccttggtagccatcttcagttccagtgtttgcttcaaatacta
agtatttgtggcctttatcttctacgtagtgaggatctctcagcgtatggttgtcgcctgagctgtagttgccttcatcgatgaactgctgtaca
ttttgatacgtttttccgtcaccgtcaaagattgatttataatcctctacaccgttgatgttcaaagagctgtctgatgctgatacgttaacttgt
gcagttgtcagtgtttgtttgccgtaatgtttaccggagaaatcagtgtagaataaacggatttttccgtcagatgtaaatgtggctgaacct

gaccattcttgtgtttggtcttttaggatagaatcatttgcatcgaatttgtcgctttctttaaagacgcggccagcgtttttccagctgtcaata gaagtttcgccgactttttgatagaacatgtaaatcgatgtgtcatccgcatttttaggatctccggctaatgcaaagacgatgtggtagcc gtgatagtttgcgacagtgccgtcagcgttttgtaatggccagctgtcccaaacgtccaggccttttgcagaagagatatttttaattgtgg acgaatcaaattcagaaacttgatatttttcatttttttgctgttcagggatttgcagcatatcatggcgtgtaatatgggaaatgccgtatgttt ccttatatggcttttggttcgtttctttcgcaaacgcttgagttgcgcctcctgccagcagtgcggtagtaaaggttaatactgttgcttgttttgc aaactttttgatgttcatcgttcatgtctcctttttttatgtactgtgttagcggtctgcttcttccagccctcctgtttgaagatggcaagttagttac gcacaataaaaaaagacctaaaatatgtaaggggtgacgccaaagtatacactttgccctttacacattttag

**SEQ ID NO: 15** (nucleotide sequence of primer $PR_{udhA}1$)
gagtacgttattcagcgccg

**SEQ ID NO: 16** (nucleotide sequence of primer $PR_{udhA}2$)
acgaatcactacgccactgt

**SEQ ID NO: 17** (nucleotide sequence of primer $PR_{udhA}3$)
gcgccatgtgctgatttacg

**SEQ ID NO: 18** (nucleotide sequence of primer $PR_{udhA}4$)
cgatattcggcacatcaggcac

## Claims

1. A process of producing an organic compound, the process comprising

   I) cultivating a genetically modified microorganism in a culture medium comprising at least one assimilable carbon source to allow the genetically modified microorganism to produce the organic compound,
   II) recovering the organic compound from the fermentation broth obtained in process step I),

   wherein the genetically modified microorganism comprises

   A) at least one genetic modification that leads to an increased activity of the enzyme encoded by the *udhA*-gene, compared to the original microorganism that has not been genetically modified,

   and wherein the original microorganism belongs to the family *Pasteurellaceae.*

2. The process according to claim 1, wherein the original microorganism belongs to the genus *Basfia.*

3. The process according to claim 2, wherein the original microorganism belongs to the species *Basfia succiniciproducens.*

4. The process according to claim 3, wherein the original microorganism is *Basfia succiniciproducens* strain DD1 as deposited under DSM 18541 with the DSMZ, Germany.

5. The process according to anyone of claims 1 to 4, wherein the comprises a nucleic acid selected from the group consisting of:

   a1) nucleic acids having the nucleotide sequence of **SEQ ID NO: 3**;
   b1) nucleic acids encoding the amino acid sequence of **SEQ ID NO: 4**;

c1) nucleic acids which are at least 70% identical to the nucleic acid of a1) or b1), the identity being the identity over the total length of the nucleic acids of a1) or b1);
d1) nucleic acids encoding an amino acid sequence which is at least 70% identical to the amino acid sequence encoded by the nucleic acid of a1) or b1), the identity being the identity over the total length of amino acid sequence encoded by the nucleic acids of a1) or b1);
e1) nucleic acids capable of hybridizing under stringent conditions with a complementary sequence of any of the nucleic acids according to a1) or b1); and
f1) nucleic acids encoding the same protein as any of the nucleic acids of a1) or b1), but differing from the nucleic acids of a1) or b1) above due to the degeneracy of the genetic code.

**6.** The process according to anyone of claims 1 to 5, wherein the at least one genetic modification A) comprises a modification of the *udhA*-gene, a modification of a regulatory element of the *udhA*-gene or a combination of both.

**7.** The process according to claim 6, wherein the at least one genetic modification A) comprises an overexpression of the *udhA*-gene.

**8.** The process according to claim 7, wherein the at least one genetic modification A) comprises an overexpression of the *udhA*-gene under control of the *pckA*-promoter.

**9.** The process according to claim 7, wherein the at least one genetic modification A) comprises an overexpression of the *udhA*-gene under control of the *gapA*-promoter.

**10.** The process according to anyone of claims 1 to 9, wherein the at least one assimilable carbon source is selected from the group consisting of sucrose, maltose, maltotriose, maltotetraose, maltopentaose, maltohexaose, maltoheptaose, D-fructose, D-glucose, D-xylose, L- arabinose, D-galactose, D-mannose, glycerol and mixtures thereof.

**11.** The process according to anyone of claims 1 to 10, wherein the organic compound is succinic acid.

**12.** The process according to anyone of claims 1 to 11, wherein process step I) is performed under unaerobic conditions.

**13.** A genetically modified microorganism that comprises

A) at least one genetic modification that leads to an increased activity of the enzyme encoded by the *udhA*-gene, compared to the original microorganism that has not been genetically modified,

wherein the original microorganism belongs to the family *Pasteurellaceae.*

**14.** The genetically modified microorganism according to claim 13, wherein the genetic modification is as defined in anyone of claims 6 to 10.

**15.** Use of a genetically modified microorganism according to claim 13 or 14 for the fermentative production of an organic compound from at least one assimilable carbon source.

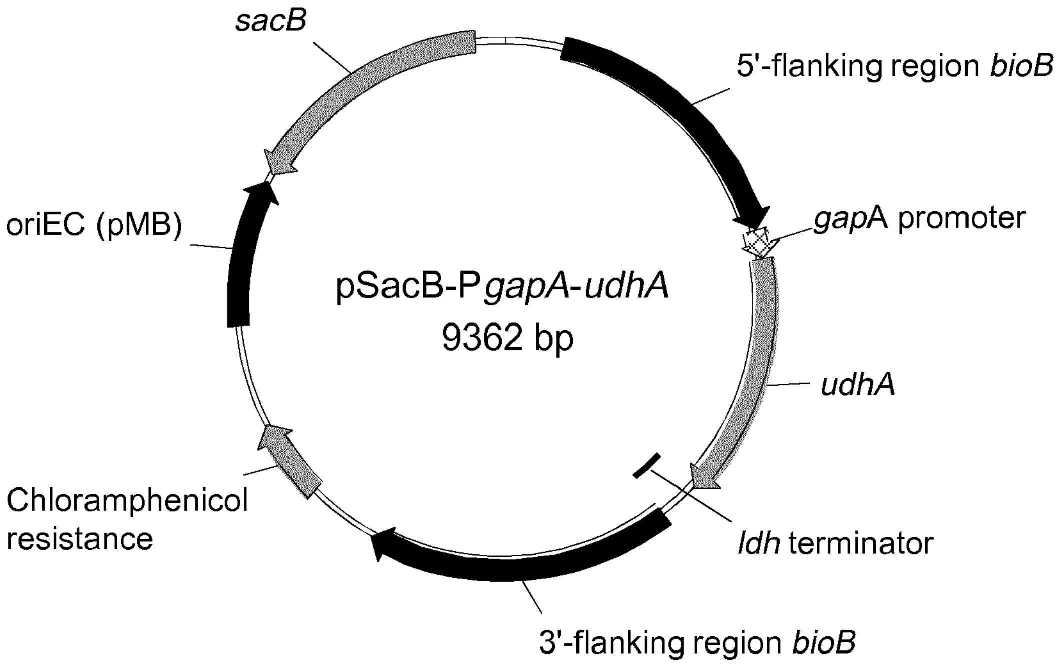

sacB
5'-flanking region bioB
gapA promoter
oriEC (pMB)
pSacB-PgapA-udhA
9362 bp
udhA
Chloramphenicol resistance
ldh terminator
3'-flanking region bioB


Fig. 1

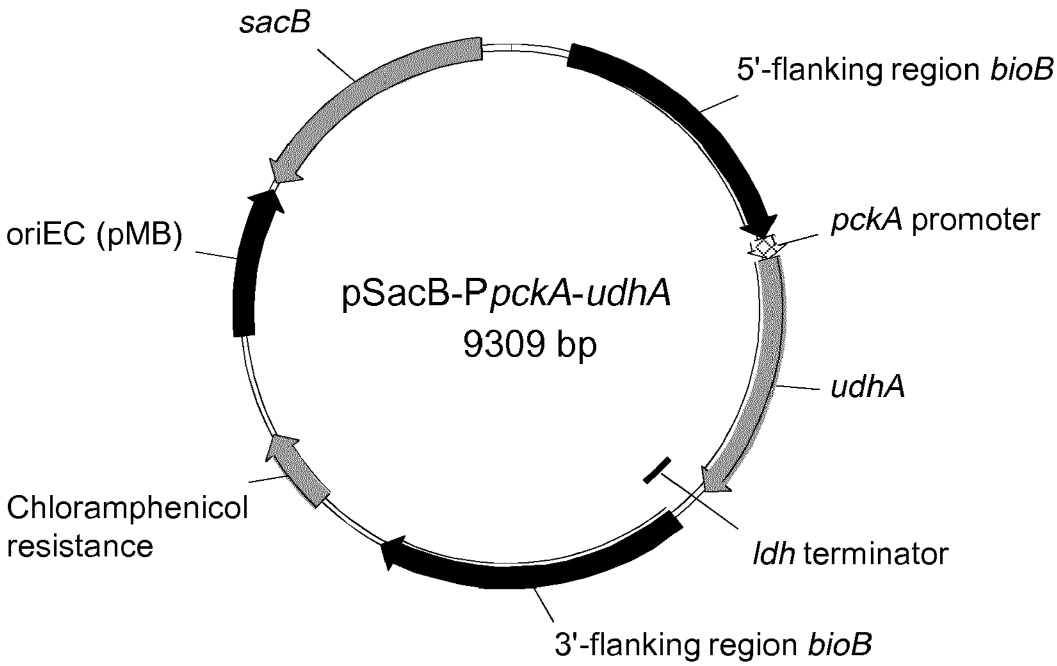
sacB
5'-flanking region bioB
pckA promoter
oriEC (pMB)
pSacB-PpckA-udhA
9309 bp
udhA
Chloramphenicol
resistance
ldh terminator
3'-flanking region bioB

Fig. 2

Europäisches Patentamt
European Patent Office
Office européen des brevets

# EUROPEAN SEARCH REPORT

Application Number
EP 17 20 9645

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2010/092155 A1 (BASF SE [DE]; SCHROEDER HARTWIG [DE]; HAEFNER STEFAN [DE]; ABENDROTH G) 19 August 2010 (2010-08-19)<br>* the whole document *<br>* in particular page 10 lines 25-30; page 12, lines 1-5;page 30 lines 10-15; claims 1-37 *<br>----- | 1-15 | INV.<br>C12N9/02<br>C12P7/46 |
| A | YUTO YAMAUCHI ET AL: "Enhanced acetic acid and succinic acid production under microaerobic conditions by Corynebacterium glutamicum harboring Escherichia coli transhydrogenase gene pntAB",<br>JOURNAL OF GENERAL AND APPLIED MICROBIOLOGY, THE,<br>vol. 60, no. 3,<br>1 January 2014 (2014-01-01), pages 112-118, XP055313131,<br>JP<br>ISSN: 0022-1260, DOI: 10.2323/jgam.60.112<br>* abstract *<br>----- | 1-15 | |
| | | | TECHNICAL FIELDS SEARCHED (IPC) |
| A | WO 2015/100338 A2 (GENOMATICA INC [US]) 2 July 2015 (2015-07-02)<br>* paragraphs [0005], [0149]; claims 42-45 *<br>-----<br>-/-- | 1-15 | C12N<br>C12P |

The present search report has been drawn up for all claims

2

| Place of search | Date of completion of the search | Examiner |
|---|---|---|
| Munich | 20 March 2018 | Dumont, Elisabeth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 20 9645

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SAUER U ET AL: "The soluble and membrane-bound transhydrogenases UdhA and PntAB have divergent functions in NADPH metabolism of Escherichia coli", JOURNAL OF BIOLOGICAL CHEMISTRY, AMERICAN SOCIETY FOR BIOCHEMISTRY AND MOLECULAR BIOLOGY, US, vol. 279, no. 8, 20 February 2004 (2004-02-20), pages 6613-6619, XP002274624, ISSN: 0021-9258, DOI: 10.1074/JBC.M311657200 * the whole document * * in particular abstract * ----- | 1-15 | |

TECHNICAL FIELDS SEARCHED (IPC)

The present search report has been drawn up for all claims

2

| Place of search | Date of completion of the search | Examiner |
|---|---|---|
| Munich | 20 March 2018 | Dumont, Elisabeth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 17 20 9645

This annex lists the patent family members relating to the patent documents cited in the above-mentioned European search report. The members are as contained in the European Patent Office EDP file on
The European Patent Office is in no way liable for these particulars which are merely given for the purpose of information.

20-03-2018

| Patent document cited in search report | Publication date | Patent family member(s) | Publication date |
|---|---|---|---|
| WO 2010092155 A1 | 19-08-2010 | BR PI1008267 A2 | 25-08-2015 |
| | | CA 2751280 A1 | 19-08-2010 |
| | | CN 102317438 A | 11-01-2012 |
| | | CN 104877935 A | 02-09-2015 |
| | | EP 2396401 A1 | 21-12-2011 |
| | | JP 5826634 B2 | 02-12-2015 |
| | | JP 2012521190 A | 13-09-2012 |
| | | KR 20110127690 A | 25-11-2011 |
| | | MY 161185 A | 14-04-2017 |
| | | US 2011300589 A1 | 08-12-2011 |
| | | US 2014127764 A1 | 08-05-2014 |
| | | US 2015197776 A1 | 16-07-2015 |
| | | WO 2010092155 A1 | 19-08-2010 |
| WO 2015100338 A2 | 02-07-2015 | CA 2935069 A1 | 02-07-2015 |
| | | CN 106062178 A | 26-10-2016 |
| | | EP 3087174 A2 | 02-11-2016 |
| | | US 2016326553 A1 | 10-11-2016 |
| | | WO 2015100338 A2 | 02-07-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2009024294 A
- WO 2010092155 A
- US 20100159543 A
- WO 2005052135 A
- EP 1005562 A
- WO 2008010373 A
- WO 2011082378 A
- WO 2011043443 A
- WO 2005030973 A
- WO 2011123268 A
- WO 2011064151 A
- EP 2360137 A
- WO 2015118111 A1

### Non-patent literature cited in the description

- **J. COOMBS.** Dictionary of Biotechnology. Stockton Press, 1994
- Quantitative Filter Hybridization. **ANDERSON ; YOUNG.** Nucleic Acid Hybridization. 1985
- Current Protocols in Molecular Biology. John Wiley & Sons, 1989, 6.3.1-6.3.6
- **YAMAUCHI et al.** Enhanced acetic acid and succinic acid production under microaerobic conditions by Corynebacterium glutamicum harboring Escherichia coli transhydrogenase gene pntAB. *J. Gen. Appl. Microbiol.,* 2014, vol. 60, 112-118
- **WONG ; MEDRANO.** Real-time PCR for mRNA quantitation. *BioTechniques,* July 2005, vol. 39 (1), 75-85
- **LEENHOUTS et al.** *Appl Env Microbiol.,* 1989, vol. 55, 394-400
- **ASANUM N. ; HINO T.** Effects of pH and Energy Supply on Activity and Amount of Pyruvate-Formate-Lyase in Streptococcus bovis. *Appl. Environ. Microbiol.,* 2000, vol. 66, 3773-3777
- **BERGMEYER, H.U. ; BERGMEYER J. ; GRASSL, M.** Methods of Enzymatic Analysis. Verlag Chemie, 1983, vol. III, 126-133
- **CHMIEL.** *Bioprozesstechnik: Einführung in die Bioverfahrenstechnik,* vol. 1
- **CHMIEL ; HAMMES ; BAILEY.** *Biochemical Engineering*
- **SONG ; LEE.** *Carbon yield" and "YP/S,* 2006
- **QUAN ; TIAN.** *PLoS ONE,* 2009, vol. 4, e6441
- **BECKER et al.** *Biotechnology and Bioengineering,* 2013, vol. 110, 3013-3023

SEQUENCE LISTING

<110>  BASF SE

<120>  Modified microorganism for improved production of succinate

<130>  B171368EP

<160>  18

<170>  PatentIn version 3.3

<210>  1
<211>  1517
<212>  DNA
<213>  Basfia succiniciproducens

<220>
<221>  MISC_FEATURE
<223>  16 S rDNA of strain DD1

<400>  1
```
tttgatcctg gctcagattg aacgctggcg gcaggcttaa cacatgcaag tcgaacggta      60

gcgggaggaa agcttgcttt ctttgccgac gagtggcgga cgggtgagta atgcttgggg     120

atctggctta tggaggggga taacgacggg aaactgtcgc taataccgcg taatatcttc     180

ggattaaagg gtgggacttt cgggccaccc gccataagat gagcccaagt gggattaggt     240

agttggtggg gtaaaggcct accaagccga cgatctctag ctggtctgag aggatgacca     300

gccacactgg aactgagaca cggtccagac tcctacggga ggcagcagtg gggaatattg     360

cacaatgggg ggaaccctga tgcagccatg ccgcgtgaat gaagaaggcc ttcgggttgt     420

aaagttcttt cggtgacgag gaaggtgttt gttttaatag gacaagcaat tgacgttaat     480

cacagaagaa gcaccggcta actccgtgcc agcagccgcg gtaatacgga gggtgcgagc     540

gttaatcgga ataactgggc gtaaagggca tgcaggcgga cttttaagtg agatgtgaaa     600

gccccgggct taacctggga attgcatttc agactgggag tctagagtac tttagggagg     660

ggtagaattc cacgtgtagc ggtgaaatgc gtagagatgt ggaggaatac cgaaggcgaa     720

ggcagcccct tgggaagata ctgacgctca tatgcgaaag cgtggggagc aaacaggatt     780

agataccctg gtagtccacg cggtaaacgc tgtcgatttg gggattgggc tttaggcctg     840

gtgctcgtag ctaacgtgat aaatcgaccg cctggggagt acggccgcaa ggttaaaact     900

caaatgaatt gacgggggcc cgcacaagcg gtggagcatg tggtttaatt cgatgcaacg     960

cgaagaacct tacctactct tgacatccag agaatcctgt agagatacgg gagtgccttc    1020

gggagctctg agacaggtgc tgcatggctg tcgtcagctc gtgttgtgaa atgttgggtt    1080

aagtcccgca acgagcgcaa cccttatcct ttgttgccag catgtaaaga tgggaactca    1140

aaggagactg ccggtgacaa accggaggaa ggtggggatg acgtcaagtc atcatggccc    1200
```

```
ttacgagtag ggctacacac gtgctacaat ggtgcataca gagggcggcg ataccgcgag   1260

gtagagcgaa tctcagaaag tgcatcgtag tccggattgg agtctgcaac tcgactccat   1320

gaagtcggaa tcgctagtaa tcgcaaatca gaatgttgcg gtgaatacgt tcccgggcct   1380

tgtacacacc gcccgtcaca ccatgggagt gggttgtacc agaagtagat agcttaacct   1440

tcgggggggg cgtttaccac ggtatgattc atgactgggg tgaagtcgta acaaggtaac   1500

cgtaggggaa cctgcgg                                                  1517


<210>  2
<211>  3008
<212>  DNA
<213>  Basfia succiniciproducens


<220>
<221>  MISC_FEATURE
<223>  23 S rDNA of strain DD1

<400>  2
agtaataacg aacgacacag gtataagaat acttgaggtt gtatggttaa gtgactaagc     60

gtacaaggtg gatgccttgg caatcagagg cgaagaagga cgtgctaatc tgcgaaaagc    120

ttgggtgagt tgataagaag cgtctaaccc aagatatccg aatggggcaa cccagtagat    180

gaagaatcta ctatcaataa ccgaatccat aggttattga ggcaaaccgg gagaactgaa    240

acatctaagt accccgagga aaagaaatca accgagatta cgtcagtagc ggcgagcgaa    300

agcgtaagag ccggcaagtg atagcatgag gattagagga atcggctggg aagccgggcg    360

gcacagggtg atagccccgt acttgaaaat cattgtgtgg tactgagctt gcgagaagta    420

gggcgggaca cgagaaatcc tgtttgaaga aggggggacc atcctccaag gctaaatact    480

cctgattgac cgatagtgaa ccagtactgt gaaggaaagg cgaaaagaac cccggtgagg    540

ggagtgaaat agaacctgaa accttgtacg tacaagcagt gggagcccgc gagggtgact    600

gcgtaccttt tgtataatgg gtcagcgact tatattatgt agcgaggtta accgaatagg    660

ggagccgaag ggaaaccgag tcttaactgg gcgtcgagtt gcatgatata gacccgaaac    720

ccggtgatct agccatgggc aggttgaagg ttgggtaaca ctaactggag gaccgaaccg    780

actaatgttg aaaaattagc ggatgacctg tggctggggg tgaaaggcca atcaaaccgg    840

gagatagctg gttctccccg aaatctattt aggtagagcc ttatgtgaat accttcgggg    900

gtagagcact gtttcggcta gggggccatc ccggcttacc aacccgatgc aaactgcgaa    960

taccgaagag taatgcatag gagacacacg gcgggtgcta acgttcgtcg tggagaggga   1020

aacaacccag accgccagct aaggtcccaa agtttatatt aagtgggaaa cgaagtggga   1080

aggcttagac agctaggatg ttggcttaga agcagccatc atttaaagaa agcgtaatag   1140

ctcactagtc gagtcggcct gcgcggaaga tgtaacgggg ctcaaatata gcaccgaagc   1200
```

tgcggcatca ggcgtaagcc tgttgggtag gggagcgtcg tgtaagcgga agaaggtggt 1260

tcgagagggc tgctggacgt atcacgagtg cgaatgctga cataagtaac gataaaacgg 1320

gtgaaaaacc cgttcgccgg aagaccaagg gttcctgtcc aacgttaatc ggggcagggt 1380

gagtcggccc ctaaggcgag gctgaagagc gtagtcgatg ggaaacgggt taatattccc 1440

gtacttgtta taattgcgat gtggggacgg agtaggttag gttatcgacc tgttggaaaa 1500

ggtcgtttaa gttggtaggt ggagcgttta ggcaaatccg gacgcttatc aacaccgaga 1560

gatgatgacg aggcgctaag gtgccgaagt aaccgatacc acacttccag gaaaagccac 1620

taagcgtcag attataataa accgtactat aaaccgacac aggtggtcag gtagagaata 1680

ctcaggcgct tgagagaact cgggtgaagg aactaggcaa aatagcaccg taacttcggg 1740

agaaggtgcg ccggcgtaga ttgtagaggt atacccttga aggttgaacc ggtcgaagtg 1800

acccgctggc tgcaactgtt tattaaaaac acagcactct gcaaacacga aagtggacgt 1860

atagggtgtg atgcctgccc ggtgctggaa ggttaattga tggcgttatc gcaagagaag 1920

cgcctgatcg aagccccagt aaacggcggc cgtaactata acggtcctaa ggtagcgaaa 1980

ttccttgtcg ggtaagttcc gacctgcacg aatggcataa tgatggccag gctgtctcca 2040

cccgagactc agtgaaattg aaatcgccgt gaagatgcgg tgtacccgcg gctagacgga 2100

aagaccccgt gaacctttac tatagcttga cactgaacct tgaattttga tgtgtaggat 2160

aggtgggagg ctttgaagcg gtaacgccag ttatcgtgga gccatccttg aaataccacc 2220

ctttaacgtt tgatgttcta acgaagtgcc cggaacgggt actcggacag tgtctggtgg 2280

gtagtttgac tggggcggtc tcctcccaaa gagtaacgga ggagcacgaa ggtttgctaa 2340

tgacggtcgg acatcgtcag gttagtgcaa tggtataagc aagcttaact gcgagacgga 2400

caagtcgagc aggtgcgaaa gcaggtcata gtgatccggt ggttctgaat ggaagggcca 2460

tcgctcaacg gataaaaggt actccgggga taacaggctg ataccgccca agagttcata 2520

tcgacggcgg tgtttggcac ctcgatgtcg gctcatcaca tcctggggct gaagtaggtc 2580

ccaagggtat ggctgttcgc catttaaagt ggtacgcgag ctgggtttaa aacgtcgtga 2640

gacagtttgg tccctatctg ccgtgggcgt tggagaattg agaggggctg ctcctagtac 2700

gagaggaccg gagtggacgc atcactggtg ttccggttgt gtcgccagac gcattgccgg 2760

gtagctacat gcggaagaga taagtgctga aagcatctaa gcacgaaact tgcctcgaga 2820

tgagttctcc cagtatttaa tactgtaagg gttgttggag acgacgacgt agataggccg 2880

ggtgtgtaag cgttgcgaga cgttgagcta accggtacta attgcccgag aggcttagcc 2940

atacaacgct caagtgtttt tggtagtgaa agttattacg gaataagtaa gtagtcaggg 3000

aatcggct 3008

<210> 3
<211> 1401
<212> DNA
<213> Escherichia coli

<220>
<221> MISC_FEATURE
<223> nucleotide sequence of udhA-gene from E. coli

<400> 3
atgccacatt cctacgatta cgatgccata gtaataggtt ccggccccgg cggcgaaggc 60

gctgcaatgg gcctggttaa gcaaggtgcg cgcgtcgcag ttatcgagcg ttatcaaaat 120

gttggcggcg gttgcaccca ctggggcacc atcccgtcga aagctctccg tcacgccgtc 180

agccgcatta tagaattcaa tcaaaaccca ctttacagcg accattcccg actgctccgc 240

tcttcttttg ccgatatcct taaccatgcc gataacgtga ttaatcaaca aacgcgcatg 300

cgtcagggat tttacgaacg taatcactgt gaaatattgc agggaaacgc tcgctttgtt 360

gacgagcata cgttggcgct ggattgcccg gacggcagcg ttgaaacact aaccgctgaa 420

aaatttgtta ttgcctgcgg ctctcgtcca tatcatccaa cagatgttga tttcacccat 480

ccacgcattt acgacagcga ctcaattctt agcatgcacc acgaaccgcg ccatgtgctg 540

atttacggtg ctggagtgat cggctgtgaa tatgcgtcga tcttccgcgg tatggatgta 600

aaagtggatc tgatcaacac ccgcgatcgc ctgctggcat ttctcgatca agagatgtca 660

gattctctct cctatcactt ctggaacagt ggcgtagtga ttcgtcacaa cgaagagtac 720

gagaagatcg aaggctgtga cgatggtgtg atcatgcatt tgaagtcggg taaaaaactg 780

aaagctgact gcctgctcta tgccaacggt cgcaccggta ataccgattc gctggcgtta 840

cagaacattg ggctggaaac tgacagtcgc ggacagctga aggtcaacag catgtatcag 900

accgcacagc cgcacgttta cgcggtgggc gacgtgattg gttatccgag cctggcatca 960

gcggcctatg accaggggcg cattgccgcg caggcgctgg taaaaggcga agccaccgcg 1020

catctgattg aagatatccc taccggcatt tacaccatcc cggaaatcag ctctgtgggc 1080

aaaaccgaac agcagctgac cgcgatgaaa gtgccatatg aagtgggccg cgcccagttt 1140

aaacatctgg cacgcgcaca aatcgtcggc atgaacgtgg gcacgctgaa aattttgttc 1200

catcgggaaa caaaagagat tctgggtatt cactgctttg gcgagcgcgc tgccgaaatt 1260

attcatatcg gtcaggcgat tatggaacag aaaggtggcg gcaacactat tgagtacttc 1320

gtcaacacca cctttaacta cccgaccatg gcggaagcct atcgggtagc tgcgttaaac 1380

ggtttaaacc gcctgtttta a 1401

<210> 4
<211> 450

<212> PRT
<213> Escherichia coli

<220>
<221> SITE
<222> (1)..(310)
<223> amino acid sequence of NAD(P)+-transhydrogenase from E. coli

<400> 4

```
Met Pro His Ser Tyr Asp Tyr Asp Ala Ile Val Ile Gly Ser Gly Pro
1               5                   10                  15

Gly Gly Glu Gly Ala Ala Met Gly Leu Val Lys Gln Gly Ala Arg Val
            20                  25                  30

Ala Val Ile Glu Arg Tyr Gln Asn Val Gly Gly Gly Cys Thr His Trp
        35                  40                  45

Gly Thr Ile Pro Ser Lys Ala Leu Arg His Ala Val Ser Arg Ile Ile
    50                  55                  60

Glu Phe Asn Gln Asn Pro Leu Tyr Ser Asp His Ser Arg Leu Leu Arg
65                  70                  75                  80

Ser Ser Phe Ala Asp Ile Leu Asn His Ala Asp Asn Val Ile Asn Gln
                85                  90                  95

Gln Thr Arg Met Arg Gln Gly Phe Tyr Glu Arg Asn His Cys Glu Ile
            100                 105                 110

Leu Gln Gly Asn Ala Arg Phe Val Asp Glu His Thr Leu Ala Leu Asp
        115                 120                 125

Cys Pro Asp Gly Ser Val Glu Thr Leu Thr Ala Glu Lys Phe Val Ile
    130                 135                 140

Ala Cys Gly Ser Arg Pro Tyr His Pro Thr Asp Val Asp Phe Thr His
145                 150                 155                 160

Pro Arg Ile Tyr Asp Ser Asp Ser Ile Leu Ser Met His His Glu Pro
                165                 170                 175

Arg His Val Leu Ile Tyr Gly Ala Gly Val Ile Gly Cys Glu Tyr Ala
            180                 185                 190

Ser Ile Phe Arg Gly Met Asp Val Lys Val Asp Leu Ile Asn Thr Arg
        195                 200                 205
```

```
Asp Arg Leu Leu Ala Phe Leu Asp Gln Glu Met Ser Asp Ser Leu Ser
    210                 215                 220

Tyr His Phe Trp Asn Ser Gly Val Val Ile Arg His Asn Glu Glu Tyr
225                 230                 235                 240

Glu Lys Ile Glu Gly Cys Asp Asp Gly Val Ile Met His Leu Lys Ser
                245                 250                 255

Gly Lys Lys Leu Lys Ala Asp Cys Leu Leu Tyr Ala Asn Gly Arg Thr
            260                 265                 270

Gly Asn Thr Asp Ser Leu Ala Leu Gln Asn Ile Gly Leu Glu Thr Asp
        275                 280                 285

Ser Arg Gly Gln Leu Lys Val Asn Ser Met Tyr Gln Thr Ala Gln Pro
    290                 295                 300

His Val Tyr Ala Val Gly Asp Val Ile Gly Tyr Pro Ser Leu Ala Ser
305                 310                 315                 320

Ala Ala Tyr Asp Gln Gly Arg Ile Ala Ala Gln Ala Leu Val Lys Gly
                325                 330                 335

Glu Ala Thr Ala His Leu Ile Glu Asp Ile Pro Thr Gly Ile Tyr Thr
            340                 345                 350

Ile Pro Glu Ile Ser Ser Val Gly Lys Thr Glu Gln Gln Leu Thr Ala
        355                 360                 365

Met Lys Val Pro Tyr Glu Val Gly Arg Ala Gln Phe Lys His Leu Ala
    370                 375                 380

Arg Ala Gln Ile Val Gly Met Asn Val Gly Thr Leu Lys Ile Leu Phe
385                 390                 395                 400

His Arg Glu Thr Lys Glu Ile Leu Gly Ile His Cys Phe Gly Glu Arg
                405                 410                 415

Ala Ala Glu Ile Ile His Ile Gly Gln Ala Ile Met Glu Gln Lys Gly
            420                 425                 430

Gly Gly Asn Thr Ile Glu Tyr Phe Val Asn Thr Thr Phe Asn Tyr Pro
        435                 440                 445

Thr Met
    450
```

<210> 5
<211> 116
<212> DNA
<213> Basfia succiniciproducens

<220>
<221> MISC_FEATURE
<223> nucleotide sequence of pckA-promoter from strain DD1

<400> 5
ttattttcat atattttcca ataatttgat ctagataaca ttttttaggt atcttttgtc 60

ctataatgcg aaacattagt ttttattatt ttttaattta ttttatgagg tgatgt 116

<210> 6
<211> 169
<212> DNA
<213> Basfia succiniciproducens

<220>
<221> MISC_FEATURE
<223> nucleotide sequence of gapA-promoter from strain DD1

<400> 6
accatttgtc aatactcatt ttgttaaatt tcatagcgtt aaatattgac atggatcaca 60

aaaaagttat ctgttatagt ttagcgcata gtctagtttc gaatataaaa gaaatcgtgc 120

tataatttcg aaatgacgga tagttgaaat aataatattt tgatgaccc 169

<210> 7
<211> 1029
<212> DNA
<213> Basfia succinicproducens

<220>
<221> MISC_FEATURE
<223> nucleotide sequence of ldhA-gene from strain DD1

<400> 7
ttgacaaaat cagtatgttt aaataaggag ctaactatga aagttgccgt ttacagtact 60

aaaaattatg atcgcaaaca tctggatttg gcgaataaaa aatttaattt tgagcttcat 120

ttctttgatt ttttacttga tgaacaaacc gcgaaaatgg cggagggcgc cgatgccgtc 180

tgtattttcg tcaatgatga tgcgagccgc ccggtgttaa caaagttggc gcaaatcgga 240

gtgaaaatta tcgctttacg ttgtgccggt tttaataatg tggatttgga ggcggcaaaa 300

gagctgggat taaaagtcgt acgggtgcct gcgtattcgc cggaagccgt tgccgagcat 360

gcgatcggat taatgctgac tttaaaccgc cgtatccata aggcttatca gcgtacccgc 420

gatgcgaatt tttctctgga aggattggtc ggttttaata tgttcggcaa aaccgccgga 480

gtgattggta cgggaaaaat cggcttggcg gctattcgca ttttaaaagg cttcggtatg 540

```
gacgttctgg cgtttgatcc ttttaaaaat ccggcggcgg aagcgttggg cgcaaaatat    600

gtcggtttag acgagcttta tgcaaaatcc catgttatca ctttgcattg cccggctacg    660

gcggataatt atcatttatt aaatgaagcg gcttttaata aaatgcgcga cggtgtaatg    720

attattaata ccagccgcgg cgttttaatt gacagccggg cggcaatcga agcgttaaaa    780

cggcagaaaa tcggcgctct cggtatggat gtttatgaaa atgaacggga tttgtttttc    840

gaggataaat ctaacgatgt tattacggat gatgtattcc gtcgcctttc ttcctgtcat    900

aatgtgcttt ttaccggtca tcaggcgttt ttaacggaag aagcgctgaa taatatcgcc    960

gatgtgactt tatcgaatat tcaggcggtt tccaaaaatg caacgtgcga aaatagcgtt   1020

gaaggctaa                                                            1029


<210>  8
<211>  342
<212>  PRT
<213>  Basfia succiniciproducens


<220>
<221>  SITE
<222>  (1)..(342)
<223>  amino acid sequence of LdhA from strain DD1

<400>  8

Met Thr Lys Ser Val Cys Leu Asn Lys Glu Leu Thr Met Lys Val Ala
1               5                   10                  15


Val Tyr Ser Thr Lys Asn Tyr Asp Arg Lys His Leu Asp Leu Ala Asn
            20                  25                  30


Lys Lys Phe Asn Phe Glu Leu His Phe Phe Asp Phe Leu Leu Asp Glu
        35                  40                  45


Gln Thr Ala Lys Met Ala Glu Gly Ala Asp Ala Val Cys Ile Phe Val
    50                  55                  60


Asn Asp Asp Ala Ser Arg Pro Val Leu Thr Lys Leu Ala Gln Ile Gly
65                  70                  75                  80


Val Lys Ile Ile Ala Leu Arg Cys Ala Gly Phe Asn Asn Val Asp Leu
                85                  90                  95


Glu Ala Ala Lys Glu Leu Gly Leu Lys Val Val Arg Val Pro Ala Tyr
            100                 105                 110


Ser Pro Glu Ala Val Ala Glu His Ala Ile Gly Leu Met Leu Thr Leu
        115                 120                 125
```

Asn Arg Arg Ile His Lys Ala Tyr Gln Arg Thr Arg Asp Ala Asn Phe
    130                 135                 140

Ser Leu Glu Gly Leu Val Gly Phe Asn Met Phe Gly Lys Thr Ala Gly
145                 150                 155                 160

Val Ile Gly Thr Gly Lys Ile Gly Leu Ala Ala Ile Arg Ile Leu Lys
                165                 170                 175

Gly Phe Gly Met Asp Val Leu Ala Phe Asp Pro Phe Lys Asn Pro Ala
            180                 185                 190

Ala Glu Ala Leu Gly Ala Lys Tyr Val Gly Leu Asp Glu Leu Tyr Ala
        195                 200                 205

Lys Ser His Val Ile Thr Leu His Cys Pro Ala Thr Ala Asp Asn Tyr
    210                 215                 220

His Leu Leu Asn Glu Ala Ala Phe Asn Lys Met Arg Asp Gly Val Met
225                 230                 235                 240

Ile Ile Asn Thr Ser Arg Gly Val Leu Ile Asp Ser Arg Ala Ala Ile
                245                 250                 255

Glu Ala Leu Lys Arg Gln Lys Ile Gly Ala Leu Gly Met Asp Val Tyr
            260                 265                 270

Glu Asn Glu Arg Asp Leu Phe Phe Glu Asp Lys Ser Asn Asp Val Ile
        275                 280                 285

Thr Asp Asp Val Phe Arg Arg Leu Ser Ser Cys His Asn Val Leu Phe
    290                 295                 300

Thr Gly His Gln Ala Phe Leu Thr Glu Glu Ala Leu Asn Asn Ile Ala
305                 310                 315                 320

Asp Val Thr Leu Ser Asn Ile Gln Ala Val Ser Lys Asn Ala Thr Cys
                325                 330                 335

Glu Asn Ser Val Glu Gly
            340

<210> 9
<211> 741
<212> DNA
<213> Basfia succiniciproducens

```
<220>
<221>  MISC_FEATURE
<223>  nucleotide sequence of pflA-gene from strain DD1

<400>  9
atgtcggttt taggacgaat tcattcattt gaaacctgcg ggacagttga cgggccggga      60

atccgcttta ttttattttt acaaggctgc ttaatgcgtt gtaaatactg ccataataga     120

gacacctggg atttgcacgg cggtaaagaa atttccgttg aagaattaat gaaagaagtg     180

gtgacctatc gccattttat gaacgcctcg ggcggcggag ttaccgcttc cggcggtgaa     240

gctattttac aggcggaatt tgtacgggac tggttcagag cctgccataa agaaggaatt     300

aatacttgct tggataccaa cggtttcgtc cgtcatcatg atcatattat tgatgaattg     360

attgatgaca cggatcttgt gttgcttgac ctgaaagaaa tgaatgaacg ggttcacgaa     420

agcctgattg gcgtgccgaa taaaagagtg ctcgaattcg caaaatattt agcggatcga     480

aatcagcgta cctggatccg ccatgttgta gtgccgggtt atacagatag tgacgaagat     540

ttgcacatgc tggggaattt cattaaagat atgaagaata tcgaaaaagt ggaattatta     600

ccttatcacc gtctaggcgc ccataaatgg gaagtactcg gcgataaata cgagcttgaa     660

gatgtaaaac cgccgacaaa agaattaatg gagcatgtta aggggttgct tgcaggctac     720

gggcttaatg tgacatatta g                                               741


<210>  10
<211>  246
<212>  PRT
<213>  Basfia succiniciproducens


<220>
<221>  SITE
<222>  (1)..(246)
<223>  amino acid of PflA from strain DD1

<400>  10

Met Ser Val Leu Gly Arg Ile His Ser Phe Glu Thr Cys Gly Thr Val
1               5                   10                  15


Asp Gly Pro Gly Ile Arg Phe Ile Leu Phe Leu Gln Gly Cys Leu Met
            20                  25                  30


Arg Cys Lys Tyr Cys His Asn Arg Asp Thr Trp Asp Leu His Gly Gly
        35                  40                  45


Lys Glu Ile Ser Val Glu Glu Leu Met Lys Glu Val Val Thr Tyr Arg
    50                  55                  60


His Phe Met Asn Ala Ser Gly Gly Gly Val Thr Ala Ser Gly Gly Glu
```

65 70 75 80

Ala Ile Leu Gln Ala Glu Phe Val Arg Asp Trp Phe Arg Ala Cys His
85 90 95

Lys Glu Gly Ile Asn Thr Cys Leu Asp Thr Asn Gly Phe Val Arg His
100 105 110

His Asp His Ile Ile Asp Glu Leu Ile Asp Asp Thr Asp Leu Val Leu
115 120 125

Leu Asp Leu Lys Glu Met Asn Glu Arg Val His Glu Ser Leu Ile Gly
130 135 140

Val Pro Asn Lys Arg Val Leu Glu Phe Ala Lys Tyr Leu Ala Asp Arg
145 150 155 160

Asn Gln Arg Thr Trp Ile Arg His Val Val Val Pro Gly Tyr Thr Asp
165 170 175

Ser Asp Glu Asp Leu His Met Leu Gly Asn Phe Ile Lys Asp Met Lys
180 185 190

Asn Ile Glu Lys Val Glu Leu Leu Pro Tyr His Arg Leu Gly Ala His
195 200 205

Lys Trp Glu Val Leu Gly Asp Lys Tyr Glu Leu Glu Asp Val Lys Pro
210 215 220

Pro Thr Lys Glu Leu Met Glu His Val Lys Gly Leu Leu Ala Gly Tyr
225 230 235 240

Gly Leu Asn Val Thr Tyr
245

<210> 11
<211> 2313
<212> DNA
<213> Basfia succiniciproducens

<220>
<221> misc_feature
<223> nucleotide sequence of pflD-gene from strain DD1

<400> 11
atggctgaat taacagaagc tcaaaaaaaa gcatgggaag gattcgttcc cggtgaatgg 60

caaaacggcg taaatttacg tgactttatc caaaaaaact atactccgta tgaaggtgac 120

gaatcattct tagctgatgc gactcctgca accagcgagt tgtggaacag cgtgatggaa 180

ggcatcaaaa tcgaaaacaa aactcacgca cctttagatt tcgacgaaca tactccgtca 240
actatcactt ctcacaagcc tggttatatc aataaagatt tagaaaaaat cgttggtctt 300
caaacagacg ctccgttaaa acgtgcaatt atgccgtacg gcggtatcaa aatgatcaaa 360
ggttcttgcg aagtttacgg tcgtaaatta gatccgcaag tagaatttat tttcaccgaa 420
tatcgtaaaa cccataacca aggcgtattc gacgtttata cgccggatat tttacgctgc 480
cgtaaatcag gcgtgttaac cggtttaccg gatgcttacg gtcgtggtcg tattatcggt 540
gactaccgtc gtttagcggt atacggtatt gattacctga tgaaagataa aaaagcccaa 600
ttcgattcat tacaaccgcg tttggaagcg ggcgaagaca ttcaggcaac tatccaatta 660
cgtgaagaaa ttgccgaaca acaccgcgct ttaggcaaaa tcaaagaaat ggcggcatct 720
tacggttacg acatttccgg ccctgcgaca aacgcacagg aagcaatcca atggacatat 780
tttgcttatc tggcagcggt taaatcacaa aacggtgcgg caatgtcatt cggtcgtacg 840
tctacattct tagatatcta tatcgaacgt gacttaaaac gcggtttaat cactgaacaa 900
caggcgcagg aattaatgga ccacttagta atgaaattac gtatggttcg tttcttacgt 960
acgccggaat acgatcaatt attctcaggc gacccgatgt gggcaaccga aactatcgcc 1020
ggtatgggct tagacggtcg tccgttggta actaaaaaca gcttccgcgt attacatact 1080
ttatacacta tgggtacttc tccggaacca aacttaacta ttctttggtc cgaacaatta 1140
cctgaagcgt tcaaacgttt ctgtgcgaaa gtatctattg atacttcctc cgtacaatac 1200
gaaaatgatg acttaatgcg tcctgacttc aacaacgatg actatgcaat cgcatgctgc 1260
gtatcaccga tggtcgtagg taaacaaatg caattcttcg gtgcgcgcgc aaacttagct 1320
aaaactatgt tatacgcaat taacggcggt atcgatgaga aaaatggtat gcaagtcggt 1380
cctaaaactg cgccgattac agacgaagta ttgaatttcg ataccgtaat cgaacgtatg 1440
gacagtttca tggactggtt ggcgactcaa tatgtaaccg cattgaacat catccacttc 1500
atgcacgata aatatgcata tgaagcggca ttgatggcgt tccacgatcg cgacgtattc 1560
cgtacaatgg cttgcggtat cgcgggtctt tccgtggctg cggactcatt atccgcaatc 1620
aaatatgcga aagttaaacc gattcgcggc gacatcaaag ataaagacgg taatgtcgtg 1680
gcctcgaatg ttgctatcga cttcgaaatt gaaggcgaat atccgcaatt cggtaacaat 1740
gatccgcgtg ttgatgattt agcggtagac ttagttgaac gtttcatgaa aaaagttcaa 1800
aaacacaaaa cttaccgcaa cgcaactccg acacaatcta tcctgactat cacttctaac 1860
gtggtatacg gtaagaaaac cggtaatact ccggacggtc gtcgagcagg cgcgccattc 1920
ggaccgggtg caaacccaat gcacggtcgt gaccaaaaag gtgcggttgc ttcacttact 1980
tctgtggcta aacttccgtt cgcttacgcg aaagacggta tttcatatac cttctctatc 2040

gtaccgaacg cattaggtaa agatgacgaa gcgcaaaaac gcaaccttgc cggtttaatg 2100

gacggttatt tccatcatga agcgacagtg gaaggcggtc aacacttgaa tgttaacgtt 2160

cttaaccgtg aaatgttgtt agacgcgatg gaaaatccgg aaaaataccc gcaattaacc 2220

attcgtgttt caggttacgc ggttcgtttc aactcattaa ctaaagagca acaacaagac 2280

gtcatcactc gtacgtttac acaatcaatg taa 2313

<210> 12
<211> 770
<212> PRT
<213> Basfia succinicproducens

<220>
<221> SITE
<222> (1)..(770)
<223> amino acid of PflD from strain DD1

<400> 12

```
Met Ala Glu Leu Thr Glu Ala Gln Lys Lys Ala Trp Glu Gly Phe Val
1               5                   10                  15

Pro Gly Glu Trp Gln Asn Gly Val Asn Leu Arg Asp Phe Ile Gln Lys
            20                  25                  30

Asn Tyr Thr Pro Tyr Glu Gly Asp Glu Ser Phe Leu Ala Asp Ala Thr
        35                  40                  45

Pro Ala Thr Ser Glu Leu Trp Asn Ser Val Met Glu Gly Ile Lys Ile
    50                  55                  60

Glu Asn Lys Thr His Ala Pro Leu Asp Phe Asp Glu His Thr Pro Ser
65                  70                  75                  80

Thr Ile Thr Ser His Lys Pro Gly Tyr Ile Asn Lys Asp Leu Glu Lys
                85                  90                  95

Ile Val Gly Leu Gln Thr Asp Ala Pro Leu Lys Arg Ala Ile Met Pro
            100                 105                 110

Tyr Gly Gly Ile Lys Met Ile Lys Gly Ser Cys Glu Val Tyr Gly Arg
        115                 120                 125

Lys Leu Asp Pro Gln Val Glu Phe Ile Phe Thr Glu Tyr Arg Lys Thr
    130                 135                 140

His Asn Gln Gly Val Phe Asp Val Tyr Thr Pro Asp Ile Leu Arg Cys
145                 150                 155                 160
```

```
Arg Lys Ser Gly Val Leu Thr Gly Leu Pro Asp Ala Tyr Gly Arg Gly
                165                 170                 175

Arg Ile Ile Gly Asp Tyr Arg Arg Leu Ala Val Tyr Gly Ile Asp Tyr
            180                 185                 190

Leu Met Lys Asp Lys Lys Ala Gln Phe Asp Ser Leu Gln Pro Arg Leu
        195                 200                 205

Glu Ala Gly Glu Asp Ile Gln Ala Thr Ile Gln Leu Arg Glu Glu Ile
    210                 215                 220

Ala Glu Gln His Arg Ala Leu Gly Lys Ile Lys Glu Met Ala Ala Ser
225                 230                 235                 240

Tyr Gly Tyr Asp Ile Ser Gly Pro Ala Thr Asn Ala Gln Glu Ala Ile
                245                 250                 255

Gln Trp Thr Tyr Phe Ala Tyr Leu Ala Ala Val Lys Ser Gln Asn Gly
            260                 265                 270

Ala Ala Met Ser Phe Gly Arg Thr Ser Thr Phe Leu Asp Ile Tyr Ile
        275                 280                 285

Glu Arg Asp Leu Lys Arg Gly Leu Ile Thr Glu Gln Gln Ala Gln Glu
    290                 295                 300

Leu Met Asp His Leu Val Met Lys Leu Arg Met Val Arg Phe Leu Arg
305                 310                 315                 320

Thr Pro Glu Tyr Asp Gln Leu Phe Ser Gly Asp Pro Met Trp Ala Thr
                325                 330                 335

Glu Thr Ile Ala Gly Met Gly Leu Asp Gly Arg Pro Leu Val Thr Lys
            340                 345                 350

Asn Ser Phe Arg Val Leu His Thr Leu Tyr Thr Met Gly Thr Ser Pro
        355                 360                 365

Glu Pro Asn Leu Thr Ile Leu Trp Ser Glu Gln Leu Pro Glu Ala Phe
    370                 375                 380

Lys Arg Phe Cys Ala Lys Val Ser Ile Asp Thr Ser Ser Val Gln Tyr
385                 390                 395                 400

Glu Asn Asp Asp Leu Met Arg Pro Asp Phe Asn Asn Asp Asp Tyr Ala
                405                 410                 415
```

Ile Ala Cys Cys Val Ser Pro Met Val Val Gly Lys Gln Met Gln Phe
420 425 430

Phe Gly Ala Arg Ala Asn Leu Ala Lys Thr Met Leu Tyr Ala Ile Asn
435 440 445

Gly Gly Ile Asp Glu Lys Asn Gly Met Gln Val Gly Pro Lys Thr Ala
450 455 460

Pro Ile Thr Asp Glu Val Leu Asn Phe Asp Thr Val Ile Glu Arg Met
465 470 475 480

Asp Ser Phe Met Asp Trp Leu Ala Thr Gln Tyr Val Thr Ala Leu Asn
485 490 495

Ile Ile His Phe Met His Asp Lys Tyr Ala Tyr Glu Ala Ala Leu Met
500 505 510

Ala Phe His Asp Arg Asp Val Phe Arg Thr Met Ala Cys Gly Ile Ala
515 520 525

Gly Leu Ser Val Ala Ala Asp Ser Leu Ser Ala Ile Lys Tyr Ala Lys
530 535 540

Val Lys Pro Ile Arg Gly Asp Ile Lys Asp Lys Asp Gly Asn Val Val
545 550 555 560

Ala Ser Asn Val Ala Ile Asp Phe Glu Ile Glu Gly Glu Tyr Pro Gln
565 570 575

Phe Gly Asn Asn Asp Pro Arg Val Asp Asp Leu Ala Val Asp Leu Val
580 585 590

Glu Arg Phe Met Lys Lys Val Gln Lys His Lys Thr Tyr Arg Asn Ala
595 600 605

Thr Pro Thr Gln Ser Ile Leu Thr Ile Thr Ser Asn Val Val Tyr Gly
610 615 620

Lys Lys Thr Gly Asn Thr Pro Asp Gly Arg Arg Ala Gly Ala Pro Phe
625 630 635 640

Gly Pro Gly Ala Asn Pro Met His Gly Arg Asp Gln Lys Gly Ala Val
645 650 655

Ala Ser Leu Thr Ser Val Ala Lys Leu Pro Phe Ala Tyr Ala Lys Asp

660 665 670

Gly Ile Ser Tyr Thr Phe Ser Ile Val Pro Asn Ala Leu Gly Lys Asp
675 680 685

Asp Glu Ala Gln Lys Arg Asn Leu Ala Gly Leu Met Asp Gly Tyr Phe
690 695 700

His His Glu Ala Thr Val Glu Gly Gly Gln His Leu Asn Val Asn Val
705 710 715 720

Leu Asn Arg Glu Met Leu Leu Asp Ala Met Glu Asn Pro Glu Lys Tyr
725 730 735

Pro Gln Leu Thr Ile Arg Val Ser Gly Tyr Ala Val Arg Phe Asn Ser
740 745 750

Leu Thr Lys Glu Gln Gln Gln Asp Val Ile Thr Arg Thr Phe Thr Gln
755 760 765

Ser Met
770

<210> 13
<211> 9362
<212> DNA
<213> artificial sequence

<220>
<223> nucleotide sequence of pSacB-PgapA-udhA

<400> 13
gtcttgcctg ctttatcagt aacaaacccg cgcgatttac ttttcgacct cattctatta 60

gactctcgtt tggattgcaa ctggtctatt ttcctctttt gtttgataga aaatcataaa 120

aggatttgca gactacgggc ctaaagaact aaaaaatcta tctgtttctt ttcattctct 180

gtattttta tagtttctgt tgcatgggca taaagttgcc tttttaatca caattcagaa 240

aatatcataa tatctcattt cactaaataa tagtgaacgg caggtatatg tgatgggtta 300

aaaaggatcg gcggccgctc gatttaaatc ccgctttccg cctgtttatc gctgttaatg 360

tcaatgggat tgttgttgct atcacgccgt ctggtatggc tgcattttac gcaaattgcc 420

aatgttatta tgaacttagg tatgttgata ttagccgtgc ccggtttaat tttagctgtc 480

gggttgtttt tactgttgca aaaaatggaa tttggtaccg cgcatttatt tgccgtcatg 540

gtgatgtgta acgcttttc ggcaatgccg tttgtgatca gaattcttgc gcccggcatg 600

aataacaata tgcagtatta cgaaaaatta tgtcaatcgc tgggtattcg gggctggcaa 660

cgttttcgcc tgatcgagca acacaaactc gcccagccga taaaatacgc ttttgcctta 720

```
gcctgtacct tatctttggg tgattttacg gcgattgcgc tattcggcag ccagcaattt     780
tcttctttgc cttatttgct ctatcaacag ttgggcagtt atcgaggaga tcaagccgcc     840
gttacggcat tggttttact tttaatgtgc ctgcttatct ttatactggt cgaaggcgct     900
aaaaattcag ataaaaacga gaacgcgaat gataaaactt gaacgggtat attttaatta     960
taaaactatg ccgatgaatt ttaatattca tattaaaccg caggagcggg tagcgattat    1020
cggtgccagc ggagcgggga aaagcacatt attaaatctt atcgccggtt ttgaacgggc    1080
ggatgacggc gaaatttggt tgaacggcgt aaaccatact tatacggaac cttatgaacg    1140
ccccgtatcc atgttgtttc aggaaaataa cttgtttacc catttaaccg ttgaacaaaa    1200
tatcgcttta gggctaaagc ccgatttaaa acttagcgcc gccgaacaat cactggttcg    1260
acaaaccgca agtgcggtcg gtttaagccg atttttagat agaaaaccca ccgcgctttc    1320
cggcgggcag aagcagcggg tggcattagc gcgttgtttg ttacgcaata agccgatttt    1380
attgctggac gaaccttttt ccgcattgga tccggcatta agagcggaaa tgctcgattt    1440
gctttcgcaa ctttgtaatg aaaaaaaact cactttgctg atcgtcactc atcaaccgag    1500
cgaactacaa ggacgaatag acagaatttt gaccgttgaa aacgggcatt ttgcaaagaa    1560
taatgatcta aaatagctcg gaaacggtag ctatgtatta gaaaacttca aatagcaaga    1620
gataaaatac cttgcaatgc gttactaaca taaggatttg agatgaaaac aacattacaa    1680
ctttattcga atacacctca tcctcaggtg gaatattggt ctgtttgtaa ggtggaggcg    1740
cttttcgaaa cgccgttttt agaattagtg catcgtgcgg ctttagttca tcgtgaaaat    1800
tttaatccgc aagttattca actctcgacc ttaatgtcaa tcaaaaccgg cggttgtccg    1860
gaagattgcg gttattgtcc ccaatccgct cgctaaacca tttgtcaata ctcattttgt    1920
taaatttcat agcgttaaat attgacatgg atcacaaaaa agttatctgt tatagtttag    1980
cgcatagtct agtttcgaat ataaaagaaa tcgtgctata atttcgaaat gacggatagt    2040
tgaaataata atattttgat gacccatgcc acattcctac gattacgatg ccatagtaat    2100
aggttccggc cccggcggcg aaggcgctgc aatgggcctg gttaagcaag gtgcgcgcgt    2160
cgcagttatc gagcgttatc aaaatgttgg cggcggttgc acccactggg gcaccatccc    2220
gtcgaaagct ctccgtcacg ccgtcagccg cattatagaa ttcaatcaaa acccacttta    2280
cagcgaccat tcccgactgc tccgctcttc ttttgccgat atccttaacc atgccgataa    2340
cgtgattaat caacaaacgc gcatgcgtca gggattttac gaacgtaatc actgtgaaat    2400
attgcaggga aacgctcgct ttgttgacga gcatacgttg gcgctggatt gcccggacgg    2460
cagcgttgaa acactaaccg ctgaaaaatt tgttattgcc tgcggctctc gtccatatca    2520
tccaacagat gttgatttca cccatccacg catttacgac agcgactcaa ttcttagcat    2580
```

gcaccacgaa ccgcgccatg tgctgattta cggtgctgga gtgatcggct gtgaatatgc 2640
gtcgatcttc cgcggtatgg atgtaaaagt ggatctgatc aacacccgcg atcgcctgct 2700
ggcatttctc gatcaagaga tgtcagattc tctctcctat cacttctgga acagtggcgt 2760
agtgattcgt cacaacgaag agtacgagaa gatcgaaggc tgtgacgatg gtgtgatcat 2820
gcatttgaag tcgggtaaaa aactgaaagc tgactgcctg ctctatgcca acggtcgcac 2880
cggtaatacc gattcgctgg cgttacagaa cattgggctg gaaactgaca gtcgcggaca 2940
gctgaaggtc aacagcatgt atcagaccgc acagccgcac gtttacgcgg tgggcgacgt 3000
gattggttat ccgagcctgg catcagcggc ctatgaccag gggcgcattg ccgcgcaggc 3060
gctggtaaaa ggcgaagcca ccgcgcatct gattgaagat atccctaccg gcatttacac 3120
catcccggaa atcagctctg tgggcaaaac cgaacagcag ctgaccgcga tgaaagtgcc 3180
atatgaagtg ggccgcgccc agtttaaaca tctggcacgc gcacaaatcg tcggcatgaa 3240
cgtgggcacg ctgaaaattt tgttccatcg ggaaacaaaa gagattctgg gtattcactg 3300
ctttggcgag cgcgctgccg aaattattca tatcggtcag gcgattatgg aacagaaagg 3360
tggcggcaac actattgagt acttcgtcaa caccaccttt aactacccga ccatggcgga 3420
agcctatcgg gtagctgcgt taaacggttt aaaccgcctg ttttaacgcc gtttatattt 3480
ttaatagcgt acctaatcgg gtacgctttt tttatgcgga aaatccatat tttctaccg 3540
cactttttct ttaaagattt atacttaagt ctgtttgatt caatttattt ggaggtttta 3600
tgcaacacat tcaactggct cccgatttaa cattcagtcg cttaattcag ggattctggc 3660
ggttaaaaag ctggcggaaa tcgccgcagg aagcaagaat cactatgcca agaagttatg 3720
tgcgtttatc cgccgggcga caaggcatgt cggaggaaat gcaggcgatg tgttttatgg 3780
cgggtgccaa ctctattttt tatggcgata aattactggt taccggcaat gcggaagaag 3840
attgcgatag actgctaatg gaaaaattgg atttagaacc ggaaaccact gaaaaccgtt 3900
acctttcgca aaatagttaa agcctgttaa taggagagaa catgtttaaa gttcagcgga 3960
tttatgattt tgaaccgatg gaaaatgatt gcgcggtatt tgtggatcgg ctttatccgc 4020
ggggcgtaaa taaagaaaaa tttgcccatt gcctatggct aaaggatgtg tgcccgagtc 4080
atgagttacg ccggttttat catgaaaatc cgcaggaaaa ttatggagaa tttgtgctcc 4140
gttatcaact ggaattaggt aacgaattgc cgcaaaaagg attgatgatg cttaaacgtc 4200
tggagaaaga acatccgcag gttattttgc tgactgccgt taaagatgtg cgacacagcc 4260
atatcccggt cttattgaaa gcgttagccg ctattgtaga atttctataa agcaaaaagt 4320
gcggtaaaat tttttcaaaa tttccaccgc actttagatt ttgcaataag attgtaatgg 4380
tgggctggca agcccaccct accgatctga cctgcggcaa gtcatatcct ggtcttattg 4440
aaagcgttag ccgatttttc ataaaaaaag ccacacaaat gtgtggctta aacagttcct 4500

aataataatc aaacagctaa gtaaatctgc aattatagtt tgtcggcgtt ttgttttaag 4560
tattttgcaa cgccttccgg gctgtctttc ataccttctt tgcctttttc ccattgagca 4620
gggcaaactt caccgtgttg ctcgtggaat tgtaaagcgt caaccatacg taacatttcg 4680
tcgatattac ggcctaacgg taaatcgttc acaacttggt gacgaactac gccgtttgca 4740
tcgattaaga aagaggcacg taatgccacg ccggcttccg gatgttcaat gccgtatgct 4800
tttgcgattt cgtgtttagt gtctgccgct aaagcatatt gaacttgacc gataccgcct 4860
tgatctaccg cggtattacg ccatgcgttg tgagtaaatt gagagtcgat tgaaacgcca 4920
accacttcaa caccgcgttt tttgaattct tcataacgat gatcaaatgc gattaattca 4980
gacgggcaaa cgaaagtaaa gtctaacgga tagaagaaaa ttaccgccgg tttaccagca 5040
atatgttgtt tgaaattgaa gttatcaaca atttcgccgt tgcctaaaac ggctgctgat 5100
gtaaagtccg gtgcttgacg tgtaactaat accatgttta atctcctaat agaataatcg 5160
aatgaatatt ttgcgtactt tcgtaaatca tcgcaaaatc aacaatttct attctaagaa 5220
aaagaagtca atttgaatag ttgattttat ctatcaaatt aattatcgtt atctaacatt 5280
aaaaaacagg aagtattatg tcaaacaaat attcacttgc aacgacgctg gttcacgccg 5340
gtcgtagcaa acgcgttagc cagggtagtg taaatccggt ggtgcaacgc gcatcttcat 5400
tggttttcga tagtatcgca gataaacgac aagcaaccgt taaccactcc ataggccgct 5460
ttcctggctt tgcttccaga tgtatgctct cctccggaga gtaccgtgac tttattttcg 5520
gcacaaatac aggggtcgat ggataaatac ggcgatagtt tcctgacgga tgatccgtat 5580
gtaccggcgg aagacaagct gcaaacctgt cagatggaga ttgatttaat ggcggatgtg 5640
ctgagagcac cgccccgtga atccgcagaa ctgatccgct atgtgtttgc ggatgattgg 5700
ccggaataaa taaagccggg cttaatacag attaagcccg tatagggtat tattactgaa 5760
taccaaacag cttacggagg acggaatgtt acccattgag acaaccagac tgccttctga 5820
ttattaatat ttttcactat taatcagaag gaataaccat gaattttacc cggattgacc 5880
tgaatacctg gaatcgcagg gaacactttg ccctttatcg tcagcagatt aaatgcggat 5940
tcagcctgac caccaaactc gatattaccg ctttgcgtac cgcactggcg gagacaggtt 6000
ataagtttta tccgctgatg atttacctga tctcccgggc tgttaatcag tttccggagt 6060
tccggatggc actgaaagac aatgaactta tttactggga ccagtcagac ccggtcttta 6120
ctgtctttca taaagaaacc gaaacattct ctgcactgtc ctgccgttat tttccggatc 6180
tcagtgagtt tatggcaggt tataatgcgg taacggcaga atatcagcat gataccagat 6240
tgtttccgca gggaaattta ccggagaatc acctgaatat atcatcatta ccgtgggtga 6300
gttttgacgg gatttaacct gaacatcacc ggaaatgatg attattttgc cccggttttt 6360

acgatggcaa agtttcagca ggaaggtgac cgcgtattat tacctgtttc tgtacaggtt 6420

catcatgcag tctgtgatgg ctttcatgca gcacggttta ttaatacact tcagctgatg 6480

tgtgataaca tactgaaata aattaattaa ttctgtattt aagccaccgt atccggcagg 6540

aatggtggct ttttttttat attttaaccg taatctgtaa tttcgtttca gactggttca 6600

ggatcactgt acgataatgc ccccgcagtt tggtaatacc cttaataaaa aagaaacagc 6660

aaagactgac agcaataaga gctcgcttgg actcctgttg atagatccag taatgacctc 6720

agaactccat ctggatttgt tcagaacgct cggttgccgc cgggcgtttt ttattggtga 6780

gaatccaagc actagcggcg cgccggccgg cccggtgtga aataccgcac agatgcgtaa 6840

ggagaaaata ccgcatcagg cgctcttccg cttcctcgct cactgactcg ctgcgctcgg 6900

tcgttcggct gcggcgagcg gtatcagctc actcaaaggc ggtaatacgg ttatccacag 6960

aatcagggga taacgcagga aagaacatgt gagcaaaagg ccagcaaaag gccaggaacc 7020

gtaaaaaggc cgcgttgctg gcgtttttcc ataggctccg cccccctgac gagcatcaca 7080

aaaatcgacg ctcaagtcag aggtggcgaa acccgacagg actataaaga taccaggcgt 7140

ttccccctgg aagctccctc gtgcgctctc ctgttccgac cctgccgctt accggatacc 7200

tgtccgcctt tctcccttcg ggaagcgtgg cgctttctca tagctcacgc tgtaggtatc 7260

tcagttcggt gtaggtcgtt cgctccaagc tgggctgtgt gcacgaaccc cccgttcagc 7320

ccgaccgctg cgccttatcc ggtaactatc gtcttgagtc caacccggta agacacgact 7380

tatcgccact ggcagcagcc actggtaaca ggattagcag agcgaggtat gtaggcggtg 7440

ctacagagtt cttgaagtgg tggcctaact acggctacac tagaaggaca gtatttggta 7500

tctgcgctct gctgaagcca gttaccttcg gaaaaagagt tggtagctct tgatccggca 7560

aacaaaccac cgctggtagc ggtggttttt ttgtttgcaa gcagcagatt acgcgcagaa 7620

aaaaaggatc tcaagaagat cctttgatct tttctacggg gtctgacgct cagtggaacg 7680

aaaactcacg ttaagggatt ttggtcatga gattatcaaa aaggatcttc acctagatcc 7740

ttttaaaggc cggccgcggc cgccatcggc attttctttt gcgtttttat ttgttaactg 7800

ttaattgtcc ttgttcaagg atgctgtctt tgacaacaga tgttttcttg cctttgatgt 7860

tcagcaggaa gctcggcgca aacgttgatt gtttgtctgc gtagaatcct ctgtttgtca 7920

tatagcttgt aatcacgaca ttgtttcctt tcgcttgagg tacagcgaag tgtgagtaag 7980

taaaggttac atcgttagga tcaagatcca tttttaacac aaggccagtt ttgttcagcg 8040

gcttgtatgg gccagttaaa gaattagaaa cataaccaag catgtaaata tcgttagacg 8100

taatgccgtc aatcgtcatt tttgatccgc gggagtcagt gaacaggtac catttgccgt 8160

tcattttaaa gacgttcgcg cgttcaattt catctgttac tgtgttagat gcaatcagcg 8220

gtttcatcac tttttttcagt gtgtaatcat cgtttagctc aatcataccg agagcgccgt 8280

ttgctaactc agccgtgcgt tttttatcgc tttgcagaag tttttgactt tcttgacgga 8340

agaatgatgt gcttttgcca tagtatgctt tgttaaataa agattcttcg ccttggtagc 8400

catcttcagt tccagtgttt gcttcaaata ctaagtattt gtggccttta tcttctacgt 8460

agtgaggatc tctcagcgta tggttgtcgc ctgagctgta gttgccttca tcgatgaact 8520

gctgtacatt ttgatacgtt tttccgtcac cgtcaaagat tgatttataa tcctctacac 8580

cgttgatgtt caaagagctg tctgatgctg atacgttaac ttgtgcagtt gtcagtgttt 8640

gtttgccgta atgtttaccg gagaaatcag tgtagaataa acggattttt ccgtcagatg 8700

taaatgtggc tgaacctgac cattcttgtg tttggtcttt taggatagaa tcatttgcat 8760

cgaatttgtc gctgtcttta aagacgcggc cagcgttttt ccagctgtca atagaagttt 8820

cgccgacttt ttgatagaac atgtaaatcg atgtgtcatc cgcattttta ggatctccgg 8880

ctaatgcaaa gacgatgtgg tagccgtgat agtttgcgac agtgccgtca gcgttttgta 8940

atggccagct gtcccaaacg tccaggcctt ttgcagaaga gatattttta attgtggacg 9000

aatcaaattc agaaacttga tatttttcat ttttttgctg ttcagggatt tgcagcatat 9060

catggcgtgt aatatgggaa atgccgtatg tttccttata tggcttttgg ttcgtttctt 9120

tcgcaaacgc ttgagttgcg cctcctgcca gcagtgcggt agtaaaggtt aatactgttg 9180

cttgttttgc aaactttttg atgttcatcg ttcatgtctc ctttttatg tactgtgtta 9240

gcggtctgct tcttccagcc ctcctgtttg aagatggcaa gttagttacg cacaataaaa 9300

aaagacctaa aatatgtaag ggtgacgcc aaagtataca ctttgccctt tacacatttt 9360

ag 9362

<210> 14
<211> 9309
<212> DNA
<213> artificial sequence

<220>
<223> nucleotide sequence of pSacB-PpckA-udhA

<400> 14
gtcttgcctg ctttatcagt aacaaacccg cgcgatttac ttttcgacct cattctatta 60

gactctcgtt tggattgcaa ctggtctatt ttcctctttt gtttgataga aaatcataaa 120

aggatttgca gactacgggc ctaaagaact aaaaaatcta tctgtttctt ttcattctct 180

gtattttta tagtttctgt tgcatgggca taaagttgcc tttttaatca caattcagaa 240

aatatcataa tatctcattt cactaaataa tagtgaacgg caggtatatg tgatgggtta 300

aaaaggatcg gcggccgctc gatttaaatc ccgctttccg cctgtttatc gctgttaatg 360

tcaatgggat tgttgttgct atcacgccgt ctggtatggc tgcattttac gcaaattgcc 420

aatgttatta tgaacttagg tatgttgata ttagccgtgc ccggtttaat tttagctgtc 480
gggttgtttt tactgttgca aaaaatggaa tttggtaccg cgcatttatt tgccgtcatg 540
gtgatgtgta acgctttttc ggcaatgccg tttgtgatca gaattcttgc gcccggcatg 600
aataacaata tgcagtatta cgaaaaatta tgtcaatcgc tgggtattcg gggctggcaa 660
cgttttcgcc tgatcgagca acacaaactc gcccagccga taaaatacgc ttttgcctta 720
gcctgtacct tatctttggg tgattttacg gcgattgcgc tattcggcag ccagcaattt 780
tcttctttgc cttatttgct ctatcaacag ttgggcagtt atcgaggaga tcaagccgcc 840
gttacggcat tggttttact tttaatgtgc ctgcttatct ttatactggt cgaaggcgct 900
aaaaattcag ataaaaacga gaacgcgaat gataaaactt gaacgggtat attttaatta 960
taaaactatg ccgatgaatt ttaatattca tattaaaccg caggagcggg tagcgattat 1020
cggtgccagc ggagcgggga aaagcacatt attaaatctt atcgccggtt ttgaacgggc 1080
ggatgacggc gaaatttggt tgaacggcgt aaaccatact tatacggaac cttatgaacg 1140
ccccgtatcc atgttgtttc aggaaaataa cttgtttacc catttaaccg ttgaacaaaa 1200
tatcgcttta gggctaaagc ccgatttaaa acttagcgcc gccgaacaat cactggttcg 1260
acaaaccgca agtgcggtcg gtttaagccg atttttagat agaaaaccca ccgcgctttc 1320
cggcgggcag aagcagcggg tggcattagc gcgttgtttg ttacgcaata agccgatttt 1380
attgctggac gaaccttttt ccgcattgga tccggcatta agagcggaaa tgctcgattt 1440
gctttcgcaa ctttgtaatg aaaaaaaact cactttgctg atcgtcactc atcaaccgag 1500
cgaactacaa ggacgaatag acagaatttt gaccgttgaa aacgggcatt ttgcaaagaa 1560
taatgatcta aaatagctcg gaaacggtag ctatgtatta gaaaacttca aatagcaaga 1620
gataaaatac cttgcaatgc gttactaaca taaggatttg agatgaaaac aacattacaa 1680
ctttattcga atacacctca tcctcaggtg gaatattggt ctgtttgtaa ggtggaggcg 1740
cttttcgaaa cgccgttttt agaattagtg catcgtgcgg ctttagttca tcgtgaaaat 1800
tttaatccgc aagttattca actctcgacc ttaatgtcaa tcaaaaccgg cggttgtccg 1860
gaagattgcg gttattgtcc ccaatccgct cgctaattat tttcatatat tttccaataa 1920
tttgatctag ataacatttt ttaggtatct tttgtcctat aatgcgaaac attagttttt 1980
attatttttt aatttatttt atgaggtgat gtatgccaca ttcctacgat tacgatgcca 2040
tagtaatagg ttccggcccc ggcggcgaag gcgctgcaat gggcctggtt aagcaaggtg 2100
cgcgcgtcgc agttatcgag cgttatcaaa atgttggcgg cggttgcacc cactggggca 2160
ccatcccgtc gaaagctctc cgtcacgccg tcagccgcat tatagaattc aatcaaaacc 2220
cactttacag cgaccattcc cgactgctcc gctcttcttt tgccgatatc cttaaccatg 2280
ccgataacgt gattaatcaa caaacgcgca tgcgtcaggg attttacgaa cgtaatcact 2340

gtgaaatatt gcagggaaac gctcgctttg ttgacgagca tacgttggcg ctggattgcc 2400

cggacggcag cgttgaaaca ctaaccgctg aaaaatttgt tattgcctgc ggctctcgtc 2460

catatcatcc aacagatgtt gatttcaccc atccacgcat ttacgacagc gactcaattc 2520

ttagcatgca ccacgaaccg cgccatgtgc tgatttacgg tgctggagtg atcggctgtg 2580

aatatgcgtc gatcttccgc ggtatggatg taaaagtgga tctgatcaac acccgcgatc 2640

gcctgctggc atttctcgat caagagatgt cagattctct ctcctatcac ttctggaaca 2700

gtggcgtagt gattcgtcac aacgaagagt acgagaagat cgaaggctgt gacgatggtg 2760

tgatcatgca tttgaagtcg ggtaaaaaac tgaaagctga ctgcctgctc tatgccaacg 2820

gtcgcaccgg taataccgat tcgctggcgt tacagaacat tgggctggaa actgacagtc 2880

gcggacagct gaaggtcaac agcatgtatc agaccgcaca gccgcacgtt tacgcggtgg 2940

gcgacgtgat tggttatccg agcctggcat cagcggccta tgaccagggg cgcattgccg 3000

cgcaggcgct ggtaaaaggc gaagccaccg cgcatctgat tgaagatatc cctaccggca 3060

tttacaccat cccggaaatc agctctgtgg gcaaaaccga acagcagctg accgcgatga 3120

aagtgccata tgaagtgggc cgcgcccagt ttaaacatct ggcacgcgca caaatcgtcg 3180

gcatgaacgt gggcacgctg aaaattttgt tccatcggga aacaaaagag attctgggta 3240

ttcactgctt tggcgagcgc gctgccgaaa ttattcatat cggtcaggcg attatggaac 3300

agaaaggtgg cggcaacact attgagtact tcgtcaacac cacctttaac tacccgacca 3360

tggcggaagc ctatcgggta gctgcgttaa acggtttaaa ccgcctgttt taacgccgtt 3420

tatattttta atagcgtacc taatcgggta cgcttttttt atgcggaaaa tccatatttt 3480

tctaccgcac tttttcttta aagatttata cttaagtctg tttgattcaa tttatttgga 3540

ggttttatgc aacacattca actggctccc gatttaacat tcagtcgctt aattcaggga 3600

ttctggcggt taaaaagctg gcggaaatcg ccgcaggaag caagaatcac tatgccaaga 3660

agttatgtgc gtttatccgc cgggcgacaa ggcatgtcgg aggaaatgca ggcgatgtgt 3720

tttatggcgg gtgccaactc tattttttat ggcgataaat tactggttac cggcaatgcg 3780

gaagaagatt gcgatagact gctaatggaa aaattggatt tagaaccgga aaccactgaa 3840

aaccgttacc tttcgcaaaa tagttaaagc ctgttaatag gagagaacat gtttaaagtt 3900

cagcggattt atgattttga accgatggaa aatgattgcg cggtatttgt ggatcggctt 3960

tatccgcggg gcgtaaataa agaaaaattt gcccattgcc tatggctaaa ggatgtgtgc 4020

ccgagtcatg agttacgccg gttttatcat gaaaatccgc aggaaaatta tggagaattt 4080

gtgctccgtt atcaactgga attaggtaac gaattgccgc aaaaaggatt gatgatgctt 4140

aaacgtctgg agaaagaaca tccgcaggtt attttgctga ctgccgttaa agatgtgcga 4200

cacagccata tcccggtctt attgaaagcg ttagccgcta ttgtagaatt tctataaagc 4260
aaaaagtgcg gtaaaatttt ttcaaaattt ccaccgcact ttagattttg caataagatt 4320
gtaatggtgg gctggcaagc ccaccctacc gatctgacct gcggcaagtc atatcctggt 4380
cttattgaaa gcgttagccg atttttcata aaaaaagcca cacaaatgtg tggcttaaac 4440
agttcctaat aataatcaaa cagctaagta aatctgcaat tatagtttgt cggcgttttg 4500
ttttaagtat tttgcaacgc cttccgggct gtctttcata ccttctttgc ctttttccca 4560
ttgagcaggg caaacttcac cgtgttgctc gtggaattgt aaagcgtcaa ccatacgtaa 4620
catttcgtcg atattacggc ctaacggtaa atcgttcaca acttggtgac gaactacgcc 4680
gtttgcatcg attaagaaag aggcacgtaa tgccacgccg gcttccggat gttcaatgcc 4740
gtatgctttt gcgatttcgt gtttagtgtc tgccgctaaa gcatattgaa cttgaccgat 4800
accgccttga tctaccgcgg tattacgcca tgcgttgtga gtaaattgag agtcgattga 4860
aacgccaacc acttcaacac cgcgtttttt gaattcttca taacgatgat caaatgcgat 4920
taattcagac gggcaaacga aagtaaagtc taacggatag aagaaaatta ccgccggttt 4980
accagcaata tgttgtttga aattgaagtt atcaacaatt tcgccgttgc ctaaaacggc 5040
tgctgatgta aagtccggtg cttgacgtgt aactaatacc atgtttaatc tcctaataga 5100
ataatcgaat gaatattttg cgtactttcg taaatcatcg caaaatcaac aatttctatt 5160
ctaagaaaaa gaagtcaatt tgaatagttg attttatcta tcaaattaat tatcgttatc 5220
taacattaaa aaacaggaag tattatgtca aacaaatatt cacttgcaac gacgctggtt 5280
cacgccggtc gtagcaaacg cgttagccag ggtagtgtaa atccggtggt gcaacgcgca 5340
tcttcattgg ttttcgatag tatcgcagat aaacgacaag caaccgttaa ccactccata 5400
ggccgctttc ctggctttgc ttccagatgt atgctctcct ccggagagta ccgtgacttt 5460
attttcggca caaatacagg ggtcgatgga taaatacggc gatagtttcc tgacggatga 5520
tccgtatgta ccggcggaag acaagctgca aacctgtcag atggagattg atttaatggc 5580
ggatgtgctg agagcaccgc cccgtgaatc cgcagaactg atccgctatg tgtttgcgga 5640
tgattggccg gaataaataa agccgggctt aatacagatt aagcccgtat agggtattat 5700
tactgaatac caaacagctt acggaggacg gaatgttacc cattgagaca accagactgc 5760
cttctgatta ttaatatttt tcactattaa tcagaaggaa taaccatgaa ttttacccgg 5820
attgacctga atacctggaa tcgcagggaa cactttgccc tttatcgtca gcagattaaa 5880
tgcggattca gcctgaccac caaactcgat attaccgctt tgcgtaccgc actggcggag 5940
acaggttata agttttatcc gctgatgatt tacctgatct cccgggctgt taatcagttt 6000
ccggagttcc ggatggcact gaaagacaat gaacttattt actgggacca gtcagacccg 6060
gtctttactg tctttcataa agaaaccgaa acattctctg cactgtcctg ccgttatttt 6120

ccggatctca gtgagtttat ggcaggttat aatgcggtaa cggcagaata tcagcatgat 6180

accagattgt ttccgcaggg aaatttaccg gagaatcacc tgaatatatc atcattaccg 6240

tgggtgagtt ttgacgggat ttaacctgaa catcaccgga aatgatgatt attttgcccc 6300

ggtttttacg atggcaaagt ttcagcagga aggtgaccgc gtattattac ctgtttctgt 6360

acaggttcat catgcagtct gtgatggctt tcatgcagca cggtttatta atacacttca 6420

gctgatgtgt gataacatac tgaaataaat taattaattc tgtatttaag ccaccgtatc 6480

cggcaggaat ggtggctttt tttttatatt ttaaccgtaa tctgtaattt cgtttcagac 6540

tggttcagga tcactgtacg ataatgcccc cgcagtttgg taatacccct aataaaaaag 6600

aaacagcaaa gactgacagc aataagagct cgcttggact cctgttgata gatccagtaa 6660

tgacctcaga actccatctg gatttgttca gaacgctcgg ttgccgccgg gcgtttttta 6720

ttggtgagaa tccaagcact agcggcgcgc cggccggccc ggtgtgaaat accgcacaga 6780

tgcgtaagga gaaaataccg catcaggcgc tcttccgctt cctcgctcac tgactcgctg 6840

cgctcggtcg ttcggctgcg gcgagcggta tcagctcact caaaggcggt aatacggtta 6900

tccacagaat caggggataa cgcaggaaag aacatgtgag caaaaggcca gcaaaaggcc 6960

aggaaccgta aaaaggccgc gttgctggcg tttttccata ggctccgccc ccctgacgag 7020

catcacaaaa atcgacgctc aagtcagagg tggcgaaacc cgacaggact ataaagatac 7080

caggcgtttc cccctggaag ctccctcgtg cgctctcctg ttccgaccct gccgcttacc 7140

ggatacctgt ccgcctttct cccttcggga agcgtggcgc tttctcatag ctcacgctgt 7200

aggtatctca gttcggtgta ggtcgttcgc tccaagctgg gctgtgtgca cgaacccccc 7260

gttcagcccg accgctgcgc cttatccggt aactatcgtc ttgagtccaa cccggtaaga 7320

cacgacttat cgccactggc agcagccact ggtaacagga ttagcagagc gaggtatgta 7380

ggcggtgcta cagagttctt gaagtggtgg cctaactacg gctacactag aaggacagta 7440

tttggtatct gcgctctgct gaagccagtt accttcggaa aaagagttgg tagctcttga 7500

tccggcaaac aaaccaccgc tggtagcggt ggtttttttg tttgcaagca gcagattacg 7560

cgcagaaaaa aaggatctca agaagatcct ttgatctttt ctacggggtc tgacgctcag 7620

tggaacgaaa actcacgtta agggattttg gtcatgagat tatcaaaaag gatcttcacc 7680

tagatccttt taaaggccgg ccgcggccgc catcggcatt ttcttttgcg tttttatttg 7740

ttaactgtta attgtccttg ttcaaggatg ctgtctttga caacagatgt ttcttgcct 7800

ttgatgttca gcaggaagct cggcgcaaac gttgattgtt tgtctgcgta gaatcctctg 7860

tttgtcatat agcttgtaat cacgacattg tttcctttcg cttgaggtac agcgaagtgt 7920

gagtaagtaa aggttacatc gttaggatca agatccattt ttaacacaag gccagttttg 7980

ttcagcggct tgtatgggcc agttaaagaa ttagaaacat aaccaagcat gtaaatatcg 8040

ttagacgtaa tgccgtcaat cgtcattttt gatccgcggg agtcagtgaa caggtaccat 8100

ttgccgttca ttttaaagac gttcgcgcgt tcaatttcat ctgttactgt gttagatgca 8160

atcagcggtt tcatcacttt tttcagtgtg taatcatcgt ttagctcaat cataccgaga 8220

gcgccgtttg ctaactcagc cgtgcgtttt ttatcgcttt gcagaagttt ttgactttct 8280

tgacggaaga atgatgtgct tttgccatag tatgctttgt taaataaaga ttcttcgcct 8340

tggtagccat cttcagttcc agtgtttgct tcaaatacta agtatttgtg gcctttatct 8400

tctacgtagt gaggatctct cagcgtatgg ttgtcgcctg agctgtagtt gccttcatcg 8460

atgaactgct gtacattttg atacgttttt ccgtcaccgt caaagattga tttataatcc 8520

tctacaccgt tgatgttcaa agagctgtct gatgctgata cgttaacttg tgcagttgtc 8580

agtgtttgtt tgccgtaatg tttaccggag aaatcagtgt agaataaacg gatttttccg 8640

tcagatgtaa atgtggctga acctgaccat tcttgtgttt ggtcttttag gatagaatca 8700

tttgcatcga atttgtcgct ttctttaaag acgcggccag cgtttttcca gctgtcaata 8760

gaagtttcgc cgactttttg atagaacatg taaatcgatg tgtcatccgc atttttagga 8820

tctccggcta atgcaaagac gatgtggtag ccgtgatagt ttgcgacagt gccgtcagcg 8880

ttttgtaatg gccagctgtc ccaaacgtcc aggccttttg cagaagagat atttttaatt 8940

gtggacgaat caaattcaga aacttgatat ttttcatttt tttgctgttc agggatttgc 9000

agcatatcat ggcgtgtaat atgggaaatg ccgtatgttt ccttatatgg cttttggttc 9060

gtttctttcg caaacgcttg agttgcgcct cctgccagca gtgcggtagt aaaggttaat 9120

actgttgctt gttttgcaaa ctttttgatg ttcatcgttc atgtctcctt ttttatgtac 9180

tgtgttagcg gtctgcttct tccagccctc ctgtttgaag atggcaagtt agttacgcac 9240

aataaaaaaa gacctaaaat atgtaagggg tgacgccaaa gtatacactt tgccctttac 9300

acattttag 9309

<210> 15
<211> 20
<212> DNA
<213> artificial sequence

<220>
<223> nucleotide sequence of primer PRudhA1

<400> 15
gagtacgtta ttcagcgccg 20

<210> 16
<211> 20
<212> DNA
<213> artificial sequence

```
<220>
<223>  nucleotide sequence of primer PRudhA2

<400>  16
acgaatcact acgccactgt                                                  20


<210>  17
<211>  20
<212>  DNA
<213>  artificial sequence

<220>
<223>  nucleotide sequence of primer PRudhA3

<400>  17
gcgccatgtg ctgatttacg                                                  20


<210>  18
<211>  22
<212>  DNA
<213>  artificial sequence

<220>
<223>  nucleotide sequence of primer PRudhA4

<400>  18
cgatattcgg cacatcaggc ac                                               22
```